(19) Europäisches Patentamt
European Patent Office
Office européen des brevets

(11) Publication number: **0 082 501**
**A1**

# (12) EUROPEAN PATENT APPLICATION

(21) Application number: **82111736.3**

(22) Date of filing: **17.12.82**

(51) Int. Cl.³: **C 07 D 471/04**
A 61 K 31/435
//C07F9/65, C07D277/42,
(C07D471/04, 221/00, 205/00)

(30) Priority: **18.12.81 JP 205735/81**
**18.12.81 JP 205736/81**

(43) Date of publication of application:
**29.06.83 Bulletin 83/26**

(84) Designated Contracting States:
**DE FR GB IT**

(71) Applicant: **KYOWA HAKKO KOGYO CO., LTD**
**Ohtemachi Bldg. Ohtemachi 1-chome Chiyoda-ku**
**Tokyo(JP)**

(72) Inventor: **Hirata, Tadashi**
**1566-315, Nara-cho Midori-ku**
**Yokohama-shi Kanagawa-ken(JP)**

(72) Inventor: **Sato, Akira**
**1880-30, Kiso-machi**
**Machida-shi Tokyo(JP)**

(72) Inventor: **Kobayashi, Shigeru**
**4-3-20, Sagamidai**
**Sagamihara-shi Kanagawa-ken(JP)**

(74) Representative: **Casalonga, Axel et al,**
**BUREAU D.A. CASALONGA OFFICE JOSSE & PETIT**
**Baaderstrasse 12-14**
**D-8000 München 5(DE)**

(54) **Beta-lactam compound and a pharmaceutical composition containing the same.**

(57) β-Lactam compounds represented by the general formula:

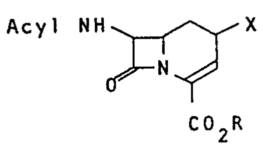

{wherein Acyl represents:

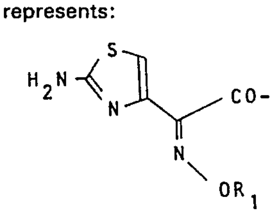

[where $R_1$ represents a hydrogen atom, a lower alkyl group having 1 to 6 carbon atoms, a substituted lower alkyl group having 1 to 6 carbon atoms (where the substituent is a cyano group, a halogen atom, a carboxyl group, a lower alkoxycarbonyl group having 2 to 6 carbon atoms, or a carbamoyl group), or a group represented by:

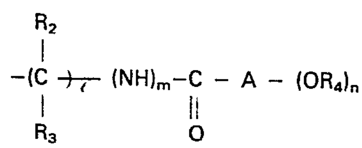

(where $R_2$ and $R_3$ are same or different, and represent hydrogen atoms, lower alkyl groups having 1 to 5 carbon atoms, or a 3 to 6-membered cycloalkylidene group with the carbon atoms bonded thereto; A represents a phenyl or naphtyl group respectively substituted by $(OR_4)_n$ and further optionally substituted by other group(s); $R_4$ represents a hydrogen atom, or a lower alkanoyl group having 1 to 5 carbon atoms; $\ell$ and m represent 0 or 1; n represents an integer of 1 to 4)], or

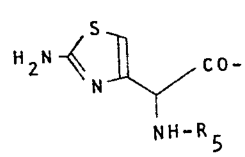

./...

EP 0 082 501 A1

[where $R_5$ represents a hydrogen atom, a lower alkanoyl group having 1 to 5 carbon atoms, a carbamoyl group, a lower alkyl group having 1 to 5 carbon atoms, a carbamoyl group substituted by a lower alkanoyl group having 1 to 5 carbon atoms, or a group represented by:

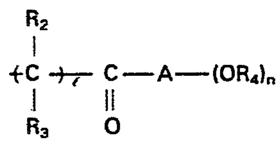

(where $R_2$, $R_3$, A, $R_4$, $\ell$ and n have the same meanings as defined above)]; X represents a methoxy group, an azide group, an amino group, $NHCOR_6$ (where $R_6$ represents a lower alkyl group having 1 to 5 carbon atoms, an amino group, or a lower alkylamino group having 1 to 5 carbon atoms, $S - R_7$ (where $R_7$ represents a substituted or unsubstituted 5 or 6-membered monocyclic or bicyclic heterocyclic group having 1 to 5 nitrogen, oxygen or sulfur atoms), a pyridinium group, or a substituted pyridinium group; and R represents a hydrogen atom, an alkali metal, an alkaline earth metal, an organic ammonium group or an ester residue readily hydrolyzable in vivo. In spite of the above definition, where X is a methoxy group, $R_1$ is a hydrogen atom, a lower alkyl group having 1 to 6 carbon atoms, or a substituted lower alkyl group having 1 to 6 carbon atoms (where the substituent is a cyano group, a halogen atom, or a carbamoyl group) } have a strong anti-microbial activities against a wide range of Gram positive and Gram negative bacteria.

# TITLE OF INVENTION

## β-LACTAM COMPOUND AND A PHARMACEUTICAL COMPOSITION CONTAINING THE SAME

## Related Applications

The present invention is related generally to the invention disclosed in U.S. Patent Application SN 419,513 filed on September 17, 1982 (or European Patent Application No. 82 108606.3), and U.S. Patent Application SN filed on September 27, 1982 (or European Patent Application No. 82 109004.0).

## Background of the Invention

The present invention relates to a β-lactam compound, more specifically to a carbacephem compound wherein an amino group at 7-position is acylated with a 2-(2-aminothiazol-4-yl)-2-syn-methoxyimino-acetyl group or 2-(2-aminothiazol-4-yl)-2-amino-acetyl group, or a related group thereof, and hydrogen atom at 4-position is substituted by a specific group, a related compound thereof, and a pharmaceutical composition containing said carbacephem compound.

Many β-lactam antibiotics have been and are under development in order to provide good chemical medicaments for curing various microbial infections. Among those compounds, carbacephem compounds wherein an amino group at 7-position is acylated with 2-(2-aminothiazol-4-yl)-2-syn-methoxyimino-acetyl group or a related group thereof, and hydrogen atom at 4-position is substituted by a bromine atom, a methyl group, an acetoxy group or a hydroxyl group, and an intermediate thereof have been known (GB 2017103A, GB 2017102A, EP 0025602A1, EP 0027882A1, EP 0014475A1, EP 0014476A1, etc.).

Further, some 2-substituted-3-cephem compounds have been known. That is, 2-methoxy substituted one is disclosed in JOC 41, 2150 (1976) and 2-azido substituted one in Japanese Published Unexamined Patent Application No. 646590/1980.

The β-lactam compounds of the present invention have a strong anti-microbial activities against a wide range of Gram positive and Gram negative bacteria.

## Detailed Description of the Invention

This invention relates to a novel β-lactam compound represented by the general formula (I):

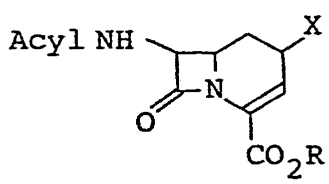

(I)

{wherein Acyl represents:

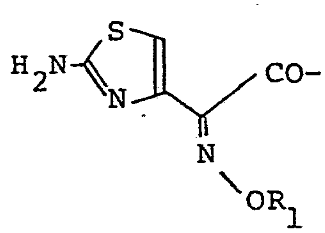

[where $R_1$ represents a hydrogen atom, a lower alkyl group having 1 to 6 carbon atoms, a substituted lower alkyl group having 1 to 6 carbon atoms (where the substituent is a cyano group, a halogen atom, a carboxyl group, a lower alkoxycarbonyl group having 2 to 6 carbom atoms, or a carbamoyl group), or a group represented by:

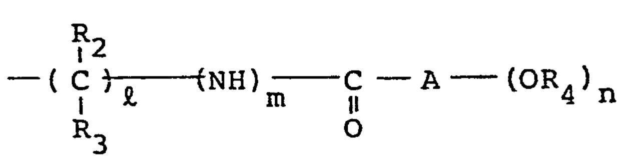

(where $R_2$ and $R_3$ are same or different, and represent hydrogen atoms, lower alkyl groups having 1 to 5 carbon atoms, or a 3 to 6-membered cycloalkylidene group with the carbon atoms bonded thereto; A represents a phenyl or naphthyl group respectively substituted by $(OR_4)_n$ and further optionally substituted by other group(s); $R_4$ represents a hydrogen atom, or a lower alkanoyl group having 1 to 5 carbon atoms; ℓ and m represent 0 or 1; n represents an integer of 1 to 4)], or

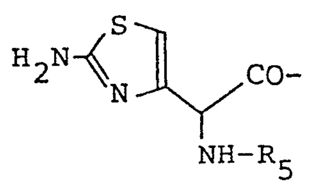

[where $R_5$ represents a hydrogen atom, a lower alkanoyl group having 1 to 5 carbon atoms, a carbamoyl group, a lower alkyl group having 1 to 5 carbon atoms, a carbamoyl group substituted by a lower alkanoyl group having 1 to 5 carbon atoms, or a group represented by:

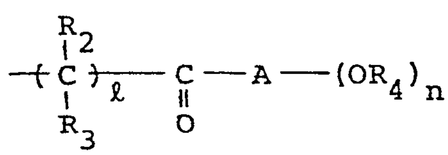

(where $R_2$, $R_3$, A, $R_4$, ℓ and n have the same meanings as defined above)]; X represents a methoxy group, an azide group, an amino group, $NHCOR_6$ (where $R_6$ represents a lower alkyl group having 1 to 5 carbon atoms, an amino group, or a lower alkylamino group having 1 to 5 carbon atoms), $S-R_7$ (where $R_7$ represents a substituted or unsubstituted 5 or 6-membered monocyclic or bicyclic heterocyclic group having 1 to 5 nitrogen, oxygen or sulfur atoms), a pyridinium group, or a substituted pyridinium group; and R represents a hydrogen atom, an alkali metal, an alkaline earth metal,

an organic ammonium group or an ester residue readily hydrolyzable in vivo. In spite of the above definition, when X is a methoxy group, $R_1$ is a hydrogen atom, a lower alkyl group having 1 to 6 carbon atoms, or a substituted lower alkyl group having 1 to 6 carbon atoms (where the substituent is a cyano group, a halogen atom, or a carbamoyl group).} (hereinafter referred to as Compound (I). The same rule is applied to a compound of other formula) and its pharmacologically acceptable acid or base addition salt.

Compound (I) and a salt thereof have an antimicrobial activity as above-mentioned.

The present invention also relates to a β-lactam compound represented by the general formula (II):

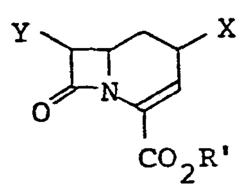

. (II)

(wherein Y represents an amino group, a phthalimido group or a phenylacetamido group, X has the same meaning as defined above, and R' has the same meaning as R or further represents a carboxyl-protecting group) and an acid addition salt thereof in case of Y being an amino group. Compound (II) is useful as an intermediate for preparation of Compound (I).

On the substituent in "substituted lower alkyl having 1 to 6 carbon atoms" in the definition of $R_1$ in the general formula (I), halogen atom includes chlorine atom, bromine atom, etc., and a lower alkoxycarbonyl having 2 to 6 carbon atoms includes methoxycarbonyl, ethoxycarbonyl, propoxycarbonyl, etc.

Further, 3 to 6 membered cycloalkylidene group in the definition of $R_2$ and $R_3$ includes cyclopropylidene, cyclobutylidene, cyclopentylidene, cyclohexylidene, etc.

The substituent on A other than $(OR_4)_n$ is exemplified by a lower alkyl group having 1 to 5 carbon atoms, a lower alkoxy group having 1 to 5 carbon atoms, an oxo group, an amino group, a nitro group, $(CH_2)_qCO_2H$, $(CH_2)_qSO_3H$, $(CH_2)_qN$ (lower alkyl group)$_2$, etc., where q represents an integer of 1 to 3 and lower alkyl group represents one having 1 to 4 carbon atoms. The lower alkylamino group having 1 to 5 carbon atoms in the definition of $R_6$ is exemplified by methylamino, ethylamino, n-propylamino, etc. Further, 5 or 6 membered monocyclic or bicyclic heterocyclic group in the definition of $R_7$ is exemplified by a furyl group, a thienyl group, a pyrrolyl group, an oxazolyl group, an isoxazolyl group, an imidazolyl group, a pyrazinyl group, a thiazolyl group, a thiazolinyl group, an isothiazolyl group, a thiadiazolyl group, a triazolyl group, a tetrazolyl group, a 1H-pyranyl group, a pyridinyl group, a pyrimidinyl group, a triazinyl group, a benzothiazolyl group, etc. The substituent thereon is exemplified by a lower alkyl group having 1 to 5 carbon atoms, an alkoxy group having 1 to 5 carbon atoms, a lower alkanoyloxy group having 1 to 5 carbon atoms, a hydroxyl group, an oxo group, an amino group, a nitro group, a phenyl group, $(CH_2)_qCO_2H$, $(CH_2)_qSO_3H$, $(CH_2)_qN$ (lower alkyl group)$_2$, etc., where q has the same definition as above and the lower alkyl group represents one having 1 to 4 carbon atoms. A substituted or unsubstituted 5 or 6 membered monocyclic or bicyclic heterocyclic group having 1 to 4 nitrogen, or 1 to 3 nitrogen and one sulfur atoms is especially preferable as

the heterocyclic group. The substituent on the substituted pyridinium group in the definition of $R_7$ is exemplified by a carbamoyl group, a carboxyl group, a cyano group, etc.

The alkali metal and the alkaline earth metal for the group R are exemplified by sodium, potassium, magnesium, calcium, barium, etc. The organic ammonium group for the group R includes ammonium groups or organic amines such as basic amino acids.

The ester residue readily dissociable in vivo in the definition of R is exemplified by a group represented by the formula:

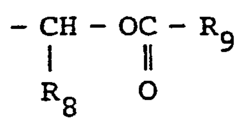

(where $R_8$ represents a hydrogen atom or a lower alkyl group having 1 to 6 carbon atoms, and $R_9$ represents a lower alkyl group having 1 to 6 carbon atoms or a phenyl group), a group represented by the formula:

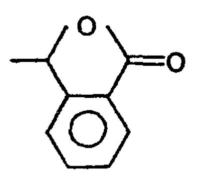

etc.

Further, the lower alkyl group in the definition of various groups in the general formula (I) is exemplified by methyl, ethyl, n-propyl, i-propyl, n-butyl, i-butyl, sec-butyl, t-butyl, n-pentyl, n-hexyl, etc. The lower alkanoyl group in the definition of various groups in the general formula (I) is exemplfied by formyl, acetyl, propionyl, n-butyryl, etc.

The carboxyl-protecting group in the definition of R' in the general formula (II) is exemplified by t-butyl, benzyl, p-nitrobenzyl, benzhydryl, trityl, trialkylsilyl such as trimethylsilyl and triethylsilyl, etc.

The pharmaceutically acceptable acid or base addition salt of Compound (I) means a salt in respect of a basic group such as an amino group, or a salt in respect of an acidic group such as a carboxyl group except of that at 2-position. As the base addition salt, the same salts as mentioned in respect of R are exemplified, and as the acid addition salt, hydrochloride, hydrobromide, nitrate, sulfate, phosphate, acetate, benzoate, maleate, fumarate, succinate, tartrate, citrate, oxalate, methanesulfonate, benzenesulfonate, etc. are exemplified. Acid addition salts of Compound (II) includes a salt with an inorganic acid or with an organic acid and are exemplified by hydrochloride, hydrobromide, phosphate, trifluoroacetate, methanesulfonate, etc.

Compound (I) can be prepared according to the following two processes.

Process A

A carbacephem compound represented by the general formula (II-1):

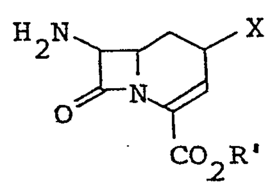

( II-1 )

(wherein X and R' have the same meanings as defined above) is reacted with a reactive derivative of a carboxylic acid represented by the general formula (III)

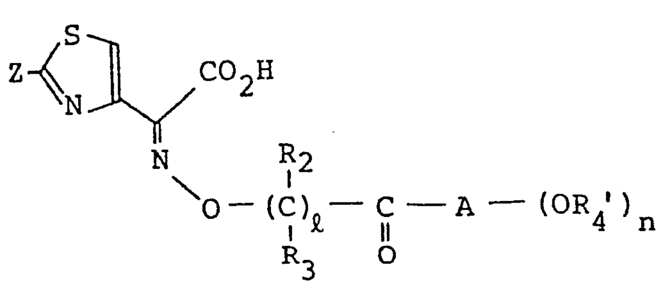

( III )

(where Z represents an amino group, or an amino group protected by a trityl group, a formyl group, a chloroacetyl group, a bromoacetyl group, a 2,2,2-trichloroacetyl group, a t-butyloxycarbonyl group, a benzyloxycarbonyl group, etc.; $R_4'$ has the same meaning as $R_4$, or further represents a hydroxyl-protecting group such as a chloroacetyl group, a bromoacetyl group, a 2,2,2-trichloroethoxycarbonyl group, a tetrahydropyranyl group, a methoxymethyl group, an ethoxyethyl group, a trimethylsilyl group and a t-butyldimethylsilyl group; $R_2$, $R_3$, A, ℓ and n have the same meanings as defined above), or by the general formula (IV):

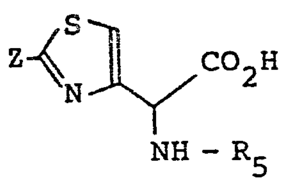

(IV)

(where Z and $R_5$ have the same meanings as defined above) to prepare a compound represented by the general formula (V):

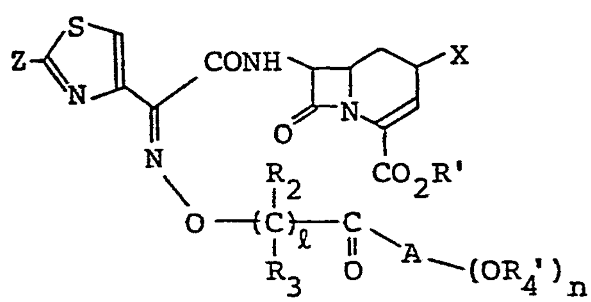

(V)

(where Z, X, R', $R_2$, $R_3$, $R_4'$, A, ℓ and n have the same meanings as defined above), or by the general formula (VI):

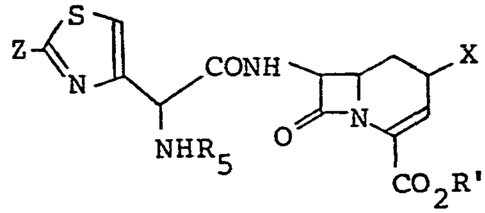

(VI)

(where Z, X, $R_5$ and R' have the same meanings as defined above), and then the resulting compound is subjected to removal of the protective group to prepare a compound represented by the general formula (I-1):

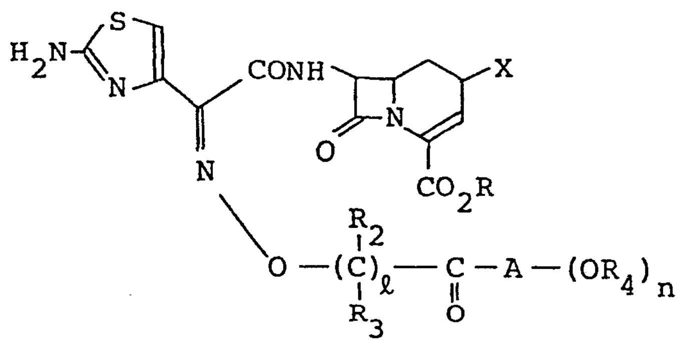

( I-1 )

(wherein X, R, $R_2$, $R_3$, $R_4$, A, ℓ and n have the same meanings as defined above) or by the general formula (I-2):

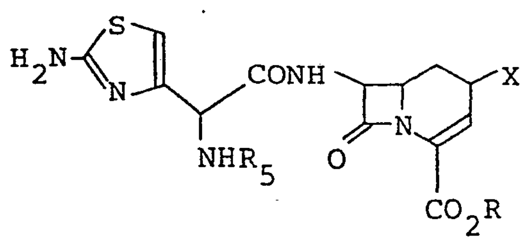

( I-2 )

(wherein X, R and $R_5$ have the same meanings as defined above). Compound (II-1), and Compounds (I-1) and (I-2) are contained respectively in Compound (II) and Compound (I).

The reactive derivative of carboxylic acid represented by the general formulae (III) and (IV) is exemplified by an acid halide such as acid chloride and acid bromide, an acid anhydride (including one formed from Compound (III) or (IV) with dicyclohexylcarbodiimide), a mixed acid anhydride such as one with a chlorocarbonic ester, e.g. methyl chlorocarbonate, ethyl chlorocarbonate, isobutyl chlorocarbonate, etc., an acid azide, an active ester such as nitrophenylthio ester and N-hydroxysuccinic acid ester, etc. The ordinary method for acylation of penicillin and cephalosporin is applicable to the reaction

using the said reactive derivative of carboxylic acid. The Compound (II-1) can be used as such, or as an inorganic salt such as a hydrochloride, a hydrobromide and a phosphate, or as an organic acid salt such as a trifluoroacetate and a methanesulfonate, or as an N-sily derivative.

The acylation reaction is carried out in an aqueous or non-aqueous solvent at a temperature of -50° to +50°C, if necessary, in the presence of a base or other acid-capturing agent. Usable as the solvent are halogenated hydrocarbons such as methylene chloride, etc., ethers such as tetrahydrofuran and dioxane, esters such as ethyl acetate, etc., amides such as dimethylformamide, etc., sulfoxides such as dimethylsulfoxide, etc., acetonitrile, acetone and water alone or in combination. Usable as the base are organic tertiary amines such as triethylamine, dimethylaniline, etc. and inorganic bases such as sodium hydrogen carbonate, potassium carbonate, etc., and usable as the other acid-capturing agent are oxirane compounds such as ethylene oxide, propylene oxide, etc.

In the step of removing the protective group to obtain Compound (I-1) or (I-2), the protective group for the functional groups (amino group, hydroxyl group and carboxyl group) in Z, $R_4'$ and R' can be removed from Compound (V) or (VI), which is an intermediate, according to the ordinary method after isolation and purification thereof, or without the isolation and purification. An appropriate method for removing the respective protective group for the hydroxyl group, amino group and carboxyl group can be applied according to the detailed description of "Protective Groups in Organic Chemistry" (1973, Plenum Press), Chapters 2, 3, 4 and 5, compiled by McOmie.

Process B

A compound represented by the general formula (VII):

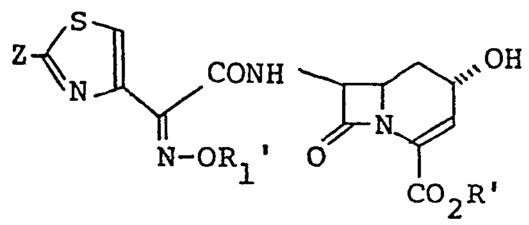

( VII )

(where Z and R' have the same meanings as defined above, and $R_1$' has the same meaning as $R_1$ or further represents $R_1$ where a carboxyl group or a hydroxyl group, when contained, is protected by a respective protecting group) is converted to a compound represented by the general formula (VIII):

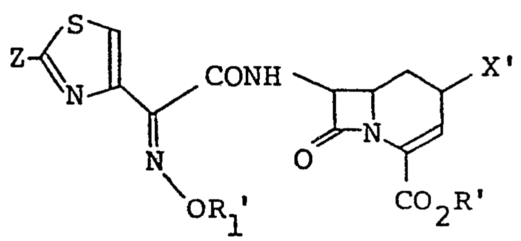

( VIII )

(where X' represents a halogen atom, a methanesulfonyloxy group, a p-toluenesulfonyloxy group, an acetoxy group or a chloroacetoxy group, and Z, R' and $R_1$' have the same meanings as defined above), which then may be isolated if desired, but is generally subjected, without isolation, to the successive substitution reaction step to obtain a compound represented by the general formula (IX):

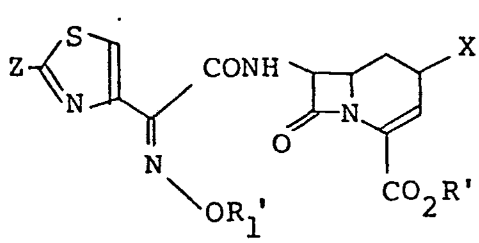

( IX )

 

(wherein Z, X, R' and $R_1'$ have the same meanings as defined above), which then may be isolated if desired, but is generally subjected, without isolation, to the successive step of removing the protective group to prepare a compound represented by the general formula (I-3):

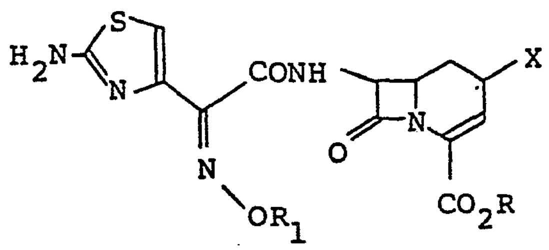

( I-3 )

(where X, R and $R_1$ have the same meanings as defined above). Compound (I-3) is contained in Compound (I).

Compound (VII) and a method for preparation thereof are disclosed in EP 0025602A1.

On $R_1'$, the carboxyl-protecting group and hydroxyl-protecting group are exemplified by the same one as exemplified respectively on R' and $R_4'$.

The preparation of Compound (VIII) can be carried out by the ordinary reagent under the ordinary reaction conditions for halogenating, mesylating, tosylating, acetylating or chloroacetylating a hydroxyl group. Particularly specific examples of the reagent include triphenylphosphine-N-bromosuccimide, triphenylphosphine-bromine, triphenylphosphine-chlorine, trialkyl phosphite or triaryl phosphite - halogen molecule, phosphorus trichloride, phosphorus tribromide, methanesulfonyl chloride-LiBr, LiCl or LiI, N-chlorosuccimidedimethyl sulfide, methanesulfonyl chloride, p-toluenesulfonyl chloride, acetyl chloride, chloroacetyl chloride, acetic anhydride,

etc. The step is carried out in an aqueous or non-aqueous solvent at a temperature of -50° to +100°C, if necessary, in the presence of a base or other acid-capturing agent. The solvent includes halogenated hydrocarbons such as methylene chloride, etc., ethers such as tetrahydrofuran and dioxane, esters such as ethyl acetate, etc., amides such as dimethylformamide, etc., sulfoxides such as dimethylsulfoxide, etc., acetonitrile, acetone or water alone or in combination. Usable as the base are organic amines such as triethylamine, dimethylaniline, pyridine, S-collidine, etc. and inorganic bases such as sodium hydrogen carbonate, etc. The same acid-capturing agents as used in the preparation of Compound (V) or (VI) are usable as other acid-capturing agents.

The substitution reaction to Compound (IX) can be carried out by reacting Compound (VIII) with a nucleophilic reagent. That is, the reaction is carried out using a heterocyclic thiol, an optionally substituted pyridine, sodium azide or methanol as the nucleophilic reagent in the presence or absence of a protic acid such as hydrogen bromide, hydrogen chloride and p-toluenesulfonic acid, or a Lewis acid such as zinc chloride, aluminum chloride, tin chloride and titanium tetrachloride. The reaction is also carried out using a heterocyclic thiol in the absence or, if necessary presence of a base such as potassium carbonate, sodium hydrogen carbonate, silver carbonate, silver oxide, sodium hydride and an organic amine, e.g. triethylamine, pyridine, etc. Similar solvents and temperature to those in the step of preparing the Compound (VIII) can be adopted in appropriate selection.

In respect of the stereochemistry of substituent X there are two types, i.e. β-form (cis to 7-acyl group) and α-form (trans to 7-acyl group), and one type can be more or substantially selectively synthesized by selecting the said reaction conditions. The step of removing the protective group can be carried out by removing the protective group for the functional groups (amino group, hydroxyl group and carboxyl group) in Z, $R_1'$ and R' according

to the ordinary procedure, thereby obtaining Compound (I-3). An appropriate method for the respective protective group for the hydroxyl group, amino group and carboxyl group is applied to the removal of the protective group. Such a method is detailedly described in "Protective Groups in Organic Chemistry" (1973, Plenum Press), Chapters 2, 3, 4 and 5, compiled by McOmie.

Compound (II) is a new intermediate for preparation of Compound (I) and can be prepared according to the following steps.

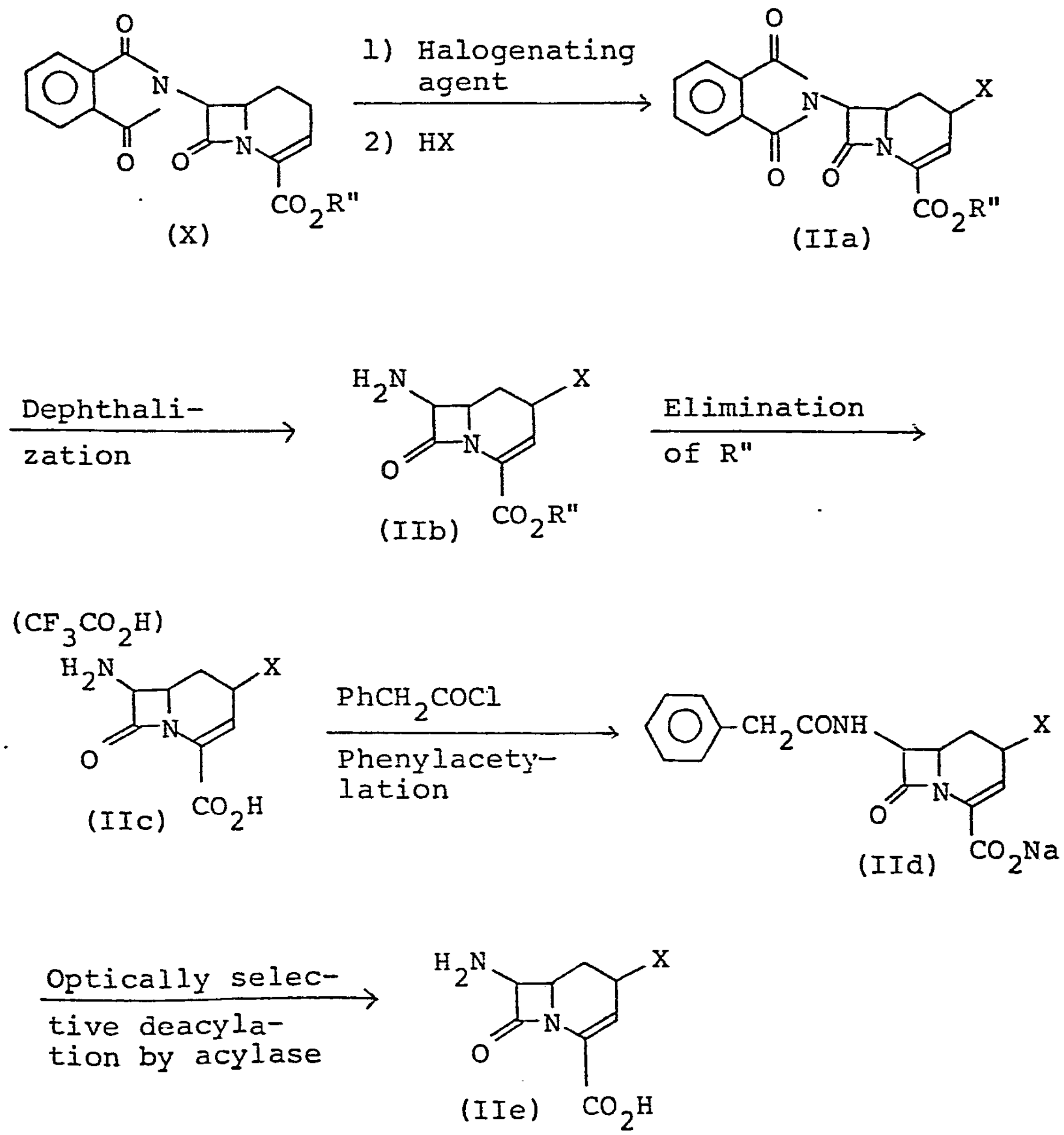

(IIa) 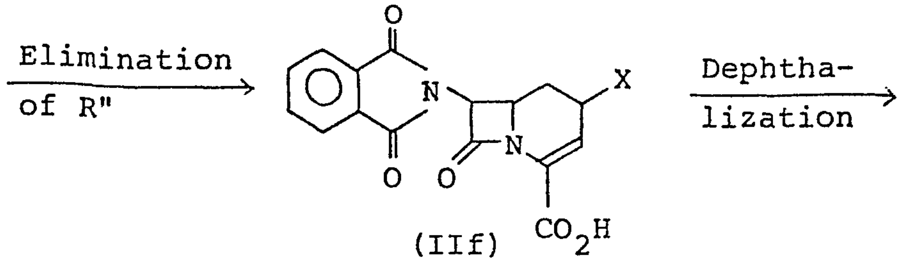

(IIf) (IIc)

PhCH$_2$COCl
Phenyl-
acetylation
(IId)
Optically
selective
deacylation
by acylase
(IIe)

(IIb) 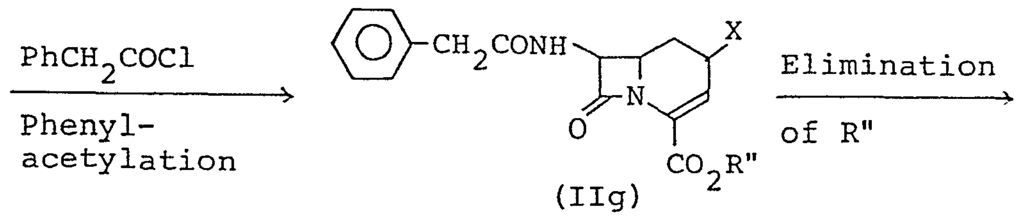

(IIg)

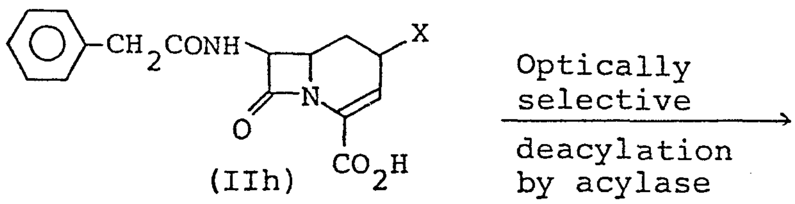

(IIh) (IIe)

(where R" represents a carboxyl-protecting group) Compounds IIa to IIh are contained in Compound (II). The same carboxyl-protecting groups as exemplified for R' are exemplified as R".

Compound IIc, IId, IIe or IIh can be reacted with a hydroxide or a carbonate of alkali metal or alkaline earth metal, an organic amine, 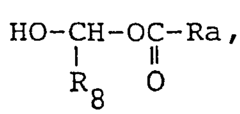, 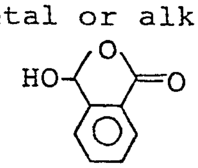, etc. according to an ordinary acid-base reaction or esterification to prepare Compound (II) or Compound (II-1) where R' is R.

Compound (X) is a starting compound and can be prepared according to a method disclosed in GB 2017102A, Japanese Published Unexamined Patent Application No. 91991/1982, etc. A specific example for preparation of a Compound (X) is also disclosed in Reference Examples.

The step (X) → (IIa) is a treatment with a halogenating agent used in halogenation of an aryl position such as N-halosuccinimide, for example, N-bromosuccinimide, etc., N-haloacetamide bromide, pyrrolidone hydrotribromide, etc. The used solvent is preferably a halogenated hydrocarbon such as dichloromethane, chloroform, carbon tetrachloride, etc. The reaction is carried out at a temperature from room temperature to the reflux temperature of the solvent to be used. A good result can be obtained when the reaction is carried out in the presence of a catalyst such as perbenzoic acid, azobisisobutyronitrile, etc. The thus obtained halogen derivative can be converted to a Compound (IIa) by reacting it with various nucleophilic agents represented by HX. The convertion is carried out using a heterocyclic thiol, an optionally substituted pyridine, sodium azide or methanol as the nucleophilic reagent in the presence or absence of a protic acid such as hydrogen bromide, hydrogen chloride and p-toluenesulfonic acid, or a Lewis acid such as zinc chloride, aluminum chloride, tin chloride and titanium tetrachloride. The conversion is also carried out using a heterocyclic thiol in the absence or, if necessary presence of a base such as potassium carbonate, sodium hydrogen carbonate, silver carbonate, silver oxide, sodium hydride and an organic amine, e.g. triethylamine, pyridine, etc. The solvent for use in the conversion includes an aprotic solvent, for example, a halogenated solvent such as methylene chloride, chloroform, and carbon tetrachloride, an ether solvent such as tetrahydrofuran, dioxane, etc., an ester solvent such as ethyl acetate, etc., an aprotic polar solvent such as dimethylformamide, dimethylsulfoxide, and hexamethylphosphoramide, acetone, acetonitrile, etc. The solvents can be used alone or in mixture. The conversion is carried out at -20°C to 100°C.

According to the stereochemistry of group X, there are two types, that is, cis type (β-form) and trans type (α-form) with respect to the 7-position phthalimido group, and it is possible to synthesize one type more than the other or to selectively synthesize substantially one type by selecting the said reaction conditions.

The dephthalization step (Ia) → (Ib) and (If) → (Ic) can be carried out according to the process disclosed in J. Am. Chem. Soc. 97 5582 (1975), which is carried out in three steps as given below, and it is also possible to carry out hydrazinolysis in one step, depending upon selection of the conditions.

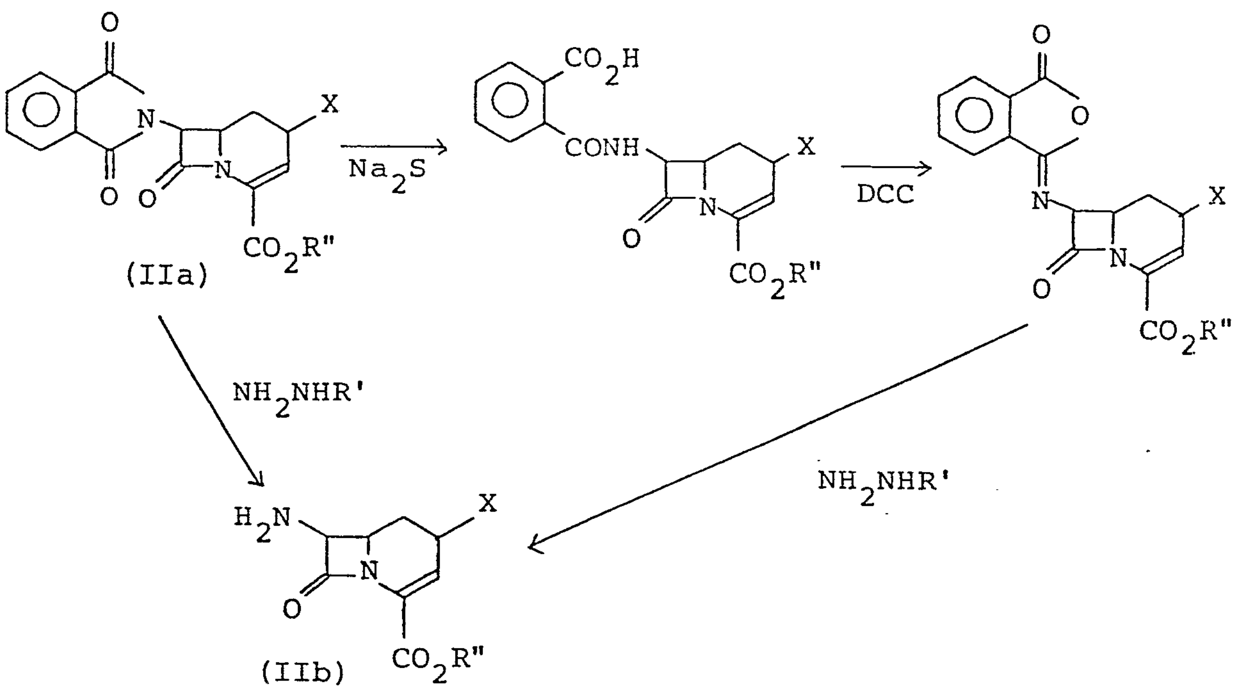


The step for elimination of R", (IIb) → (IIc), (IIa) → (IIf) or (IIg) → (IIh) can be carried out according to the process disclosed in detail in "Protective Groups in Organic Chemistry" compiled by McOmie (1973, Plenum Press), Chapter 5. Particularly preferable acid includes trifluoroacetic acid, formic acid, hydrochloric acid-acetic acid, hydrogen chloride, etc. The solvent includes ethyl acetate, methylene chloride, anisol, etc. and can be used alone or in mixture. The reaction can be carried out in

the absence of the solvent. The reaction is carried out at a temperature from -10°C to 60°C.

The phenylacetylation step (IIc) → (IId) and (IIb) → (IIg) can be carried out by acylation reaction according to the ordinary acid chloride process using phenylacetyl chloride, where aqueous tetrahydrofuran, aqueous acetone, aqueous dioxane, etc. are used as a solvent, and sodium hydrogen carbonate, potassium carbonate, triethylamine, etc. are used as a base, and the reaction is carried out at a temperature from -10° to 35°C.

The optically selective deacylation step by acylase (IId) → (IIe) and (IIh) → (IIe) can be carried out with a microorganism, cultured product of the microorganism and/or the enzyme produced by the microorganism, which can optically selectively deacylate Compound (IId) or (IIh), thereby producing the optically active Compound (IIe). The step can be carried out according to the processes disclosed, for example, in EP 0027882A1, EP 0025602A1, etc.

The present β-lactam compounds (I) have strong antimicrobial activities against a wide range of Gram positive and Gram negative bacteria and are useful for curing various infections, as a bactericide or as a component of a disinfectant.

Thus, the invention includes within its scope pharmaceutical compositions comprising, as an active ingredient, a β-lactam Compound (I) or a pharmaceutically acceptable salt thereof in association with a pharmaceutical carrier or diluent. The compounds of this invention are administered by parenteral (intramuscular, intraperitoneal, intravenous or subcutaneous injection routes), oral or rectal route of administration and can be formulated in dosage forms appropriate for each route of administration.

Preparations according to this invention for parenteral administration include sterile aqueous or non-aqueous solutions, suspensions or emulsions. Examples of non-aqueous solvents or vehicles are propylene glycol, polyethylene glycol, vegetable oils such as olive oil, and

injectable organic esters such as ethyl oleate. Such dosage forms may also contain adjuvants such as preserving, wetting, emulsifying and dispersing agents. They may be sterilized by, for example, filtration through a bacteria-retaining filter, by incorporating sterilizing agents into the compositions, by irradiating the compositions or by heating the compositions. They can also be manufactured in the form of sterile solid compositions which can be dissolved in sterile water or some other sterile injectable medium immediately before use.

Compositions for oral administration may be presented in a form suitable for absorption by the gastrointestinal tract. Tablets and capsules for oral administration may be in unit dose presentation form, and may contain conventional excipients such as binding agents, for example, syrup, acacia, gelatin, sorbitol, tragacanth or polyvinylpyrrolidone; fillers, for example, lactose, sugar, maize-starch, calcium phosphate, sorbitol or glycine; lubricants, for example, magnesium stearate, talc, polyethylene glycol, silica; disintegrants, for example, potato starch or acceptable wetting agent such as sodium lauryl sulphate. The tablets may be coated according to methods well known in the arts. Oral liquid preparations may be in the form of aqueous or oily suspension, solution, emulsions, syrups, etc., or may be presented as a dry product, for reconstitution with water or other suitable vehicle before use. Such liquid preparations may contain conventional additive such as suspending agents, for example, sorbitol syrup, methyl cellulose, glucose sugar syrup, gelatin, hydroxyethylcellulose, carboxymethylcellulose, aluminum stearate gel, emulsifying agents, for example, lecithin or sorbitan monooleate; non-aqueous vehicles, which may include edible oils, for example, almond oil, coconut oil, propylene glycol or ethyl alcohol; preservatives, for example, methyl or propyl p-hydroxybenzoates or sorbic acid.

Compositions for rectal administration are preferably suppositories which may contain, in addition to

the active substance, excipients such as cocoa butter or a suppository wax.

The dosage of active ingredient in the compositions of this invention may be varied; however, it is necessary that the amount of the active ingredient shall be such that a suitable dosage form is obtained. The selected dosage depends upon the desired therapeutic effect, on the route of administration, and on the duration of the treatment. Generally, dosage levels of between 5 and 350 mg/kg of body weight daily are administered to mammalian patients to achieve an antibiotic effect.

Examples of the present invention are given below:

Example 1

Preparation of (±)-cis-7β-[2-(2-amino-4-thiazolyl)-2-syn-methoxyimino-acetamido]-4β-(1-methyl-1,2,3,4-tetrazol-5-yl)-thio-1-azabicyclo[4,2,0]oct-2-en-8-oxo-2-carboxylic acid:

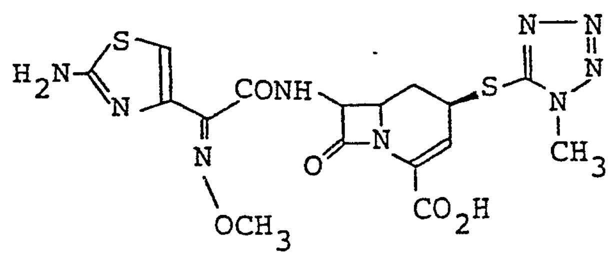

At first, 112 mg of 2-(2-tritylamino-4-thiazolyl)-2-syn-methoxyiminoacetic acid is dissolved in 3 ml of anhydrous tetrahydrofuran, and 36 μl of triethylamine is added thereto under ice cooling. Then, 53 mg of phosphorus pentachloride is added thereto, and the mixture is stirred at the same temperature for one hour to obtain an acid chloride solution.

On the other hand, 50 mg of (±)-cis-7β-amino-4β-(1-methyl-1,2,3,4-tetrazol-5-yl)-thio-1-azabicyclo-[4,2,0]oct-2-en-8-oxo-2-carboxylic acid trifluoroacetate, obtained according to the process of Example 33, which is hereinafter referred to as "Compound a", is dissolved in a mixture of 4 ml of water and 2 ml of tetrahydrofuran, and while triethylamine is added thereto under ice cooling and with stirring to keep the solution at a pH of 7.5 to 8.0, the said acid chloride solution is added dropwise thereto. Then, the mixture is subjected to reaction at the same temperature for two hours. Then, the mixture is adjusted to pH 2 with 1N hydrochloric acid, and a saturated aqueous sodium chloride solution is added thereto. The mixture is extracted three times with ethyl acetate. The organic layer is washed once with a saturated aqueous sodium chloride solution, and the solvent is distilled off

under reduced pressure. Then, 8 ml of an aqueous 50% acetic acid solution is added to the residue, and the mixture is stirred at 50°C for one hour and then cooled to room temperature. The deposited white precipitate is removed by filtration. The reaction solution is concentrated, and the residue is dissolved in a small amount of dimethylsulfoxide. The solution is subjected to adsorption on 20 ml of Diaion HP 20 AG resin (made by Mitsubishi Kasei Kogyo Co., Ltd., Japan) in a column. Fractions containing the desired compound, which are eluted with water-methanol (2 : 3), are concentrated under reduced pressure to obtain 11 mg of the desired compound as powder. Yield: 18.8%

NMR($d_6$DMSO-$CD_3$OD)δ: 1.76-2.71(2H, m), 3.90(3H, s), 4.00(3H, s), 5.53(1H, d, J = 5.4Hz), 6.37(1H, d, J = 2.4Hz), 6.84(1H, s)

IR $\nu_{max}^{cm^{-1}}$ (KBr): 1790(sh), 1780(sh), 1775, 1770(sh), 1665, 1630

<u>Example 2</u>

Preparation of (±)-cis-7β-[2-(2-amino-4-thiazolyl)-2-syn-methoxyimino-acetamido]-4α-(1-methyl-1,2,3,4-tetrazol-5-yl)-thio-1-azabicyclo[4,2,0]oct-2-en-8-oxo-2-carboxylic acid:

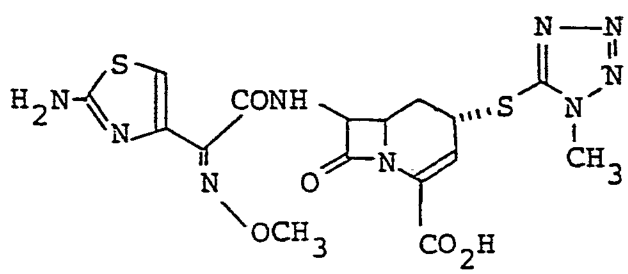

The same procedure as in Example 1 is repeated using 50 mg of (±)-cis-7β-amino-4α-(1-methyl-1,2,3,4-tetrazol-5-yl)-thio-1-azabicyclo[4,2,0]oct-2-en-8-oxo-2-carboxylic acid obtained according to the process of Example 36 in place of Compound a, whereby 19.3 mg of the desired compound is obtained as powder.

Yield: 23.9%.

NMR($CD_3OD$) δ: 1.92 - 2.72(2H, m), 3.97(3H, s), 3.99 (3H, s), 5.66(1H, d, J = 5.4Hz), 6.49(1H, d, J = 5.6Hz), 6.84(1H, s)

IR $\nu_{max}^{cm^{-1}}$ (KBr): 1790(sh), 1780(sh), 1775, 1620 - 1680

Example 3

Preparation of (±)-cis-7β-[2-(2-amino-4-thiazolyl)-2-syn-methoxyiminoacetamido]-4β-azido-1-azabicyclo[4,2,0]-oct-2-en-8-oxo-2-carboxylic acid:

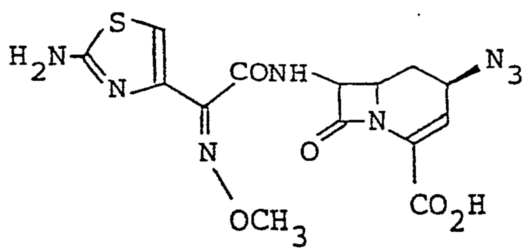

The same procedure as in Example 1 is repeated using 150 mg of (±)-cis-7β-amino-4β-azido-1-azabicyclo-[4,2,0]oct-2-en-8-oxo-2-carboxylic acid trifluoroacetic acid obtained according to the process of Example 39 in place of Compound a. Purification is carried out with 40 ml of Diaion HP 10 resin (made by Mitsubishi Kasei Kogyo Co., Ltd., Japan), and fractions containing the desired compound, which are eluted with water-methanol (1 : 1) are concentrated under reduced pressure, whereby 116 mg of the desired compound is obtained as powder. Yield: 64.1%.

NMR($d_6$DMSO-$D_2$O) δ: 1.5 - 2.4(2H, m), 3.91(3H, s), 4.06(1H, m), 4.45(1H, m), 5.41(1H, d, J = 5.4Hz), 6.16(1H, d, J = 2.0Hz), 6.83(1H, s)

IR $\nu_{max}^{cm^{-1}}$ (KBr): 2100, 1785(sh), 1775, 1765(sh), 1620 - 1680

Example 4

Preparation of disodium (±)-cis-7β-[2-(2-amino-4-thiazolyl)-2-syn-(2-carboxypropyl-2-oxyimino) acetamido]-4-β-azido-1-azabicyclo[4,2,0]oct-2-en-8-oxo-2-carboxylate:

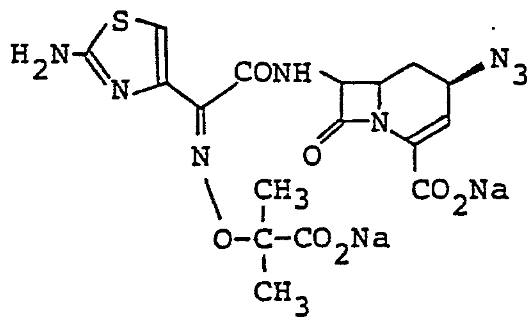

At first, 167 mg of 2-(2-tritylamino-4-thiazolyl)-2-syn-(2-tert-butoxycarbonylpropyl-2-oxyimino)acetic acid is added to 4 ml of an anhydrous methylene chloride solution containing 100 mg of phosphorus pentachloride under ice cooling. The mixture is stirred at the same temperature for 30 minutes. Then, 134 μl of triethylamine is added thereto, and the mixture is then further stirred for 5 minutes. The reaction mixture is concentrated under reduced pressure, and 4 ml of anhydrous tetrahydrofuran is added to the residue to obtain an acid chloride solution.

On the other hand, 98 mg of (±)-cis-7β-amino-4β-azido-1-azabicyclo[4,2,0]oct-2-en-8-oxo-2-carboxylic acid trifluoroacetate obtained according to the process of Example 39, is dissolved in a mixture of 5 ml of water and 5 ml of tetrahydrofuran, and while triethylamine is added thereto under ice cooling and with stirring to keep the solution at a pH of 7.5 to 8.0, the said acid chloride solution is added dropwise thereto. Then, the mixture is subjected to reaction at room temperature for one hour. Then, the mixture is adjusted to pH 2 with 1N hydrochloric acid, a saturated aqueous sodium chloride solution is added thereto, and the mixture is extracted three times with ethyl acetate. The organic layer is washed once with a saturated aqueous sodium chloride solution, and

the solvent is distilled off under reduced pressure. Then, 4 ml of anhydrous methylene chloride and 4 ml of trifluoroacetic acid are added to the residue, and the mixture is stirred at room temperature for 30 minutes. The reaction mixture is subjected to distillation under reduced pressure, and 5 ml of ethyl acetate is added to the residue. The mixture is again concentrated. This procedure is repeated twice. Then, 10 ml of an aqueous 50% acetic acid solution is added to the residue, and the mixture is stirred at 50°C for one hour. Then, the reaction mixture is concentrated under reduced pressure, and the residue is dissolved in a small amount of dimethylsulfoxide. The solution is subjected to adsorption on 20 ml of Diaion HP 20 AG resin in a column, and fractions containing the desired compound, which are eluted with water-methanol (1 : 1), are concentrated under reduced pressure, whereby 32 mg of the desired compound is obtained as powder. Yield: 21.0%.

NMR($D_2O$) δ: 1.57(6H, s), 1.57 - 2.66(2H, m), 4.15 - 4.79 (2H, m), 5.50(1H, d, J = 5.1Hz), 6.06(1H, d, J = 2.0Hz), 7.03(1H, s)

IR $\nu_{max}^{cm^{-1}}$ (KBr): 2100, 1770(sh), 1765, 1760(sh), 1600 - 1680

Example 5

Preparation of [4R, 6R, 7S]-7-[2-(2-amino-4-thiazolyl)-2-syn-methoxyiminoacetamido]-4-azido-1-azabicyclo [4,2,0]oct-2-en-8-oxo-2-carboxylic acid:

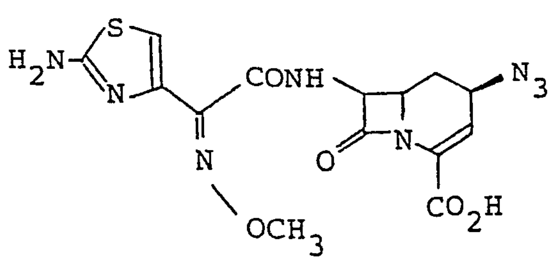

The same procedure as in Example 1 is repeated using 15 mg of [4R,6R,7S]-7-amino-4-azido-1-azabicyclo

[4,2,0]oct-2-en-8-oxo-2-carboxylic acid obtained according to the process of Example 41 in place of Compound a. Purification is carried out with 15 ml of Diaion HP-20 AG resin, and fractions containing the desired compound, which are eluted with water-methanol (4 : 3), are concentrated under reduced pressure, whereby 13 mg of the desired compound is obtained as powder. Yield: 47.6%.

$[\alpha]_D^{22}$ = +76.2° (c = 0.17, 50% aqueous methanol)

IR and NMR spectra are the same as those of the dl compound (Example 3).

Example 6

Preparation of [4R,6R,7S]-7-[2-(2-amino-4-thiazolyl)-2-syn-(3,4-diacetoxy)-benzoyloxyimino-acetamido]-4-azido-1-azabicyclo[4,2,0]oct-2-en-8-oxo-2-carboxylic acid:

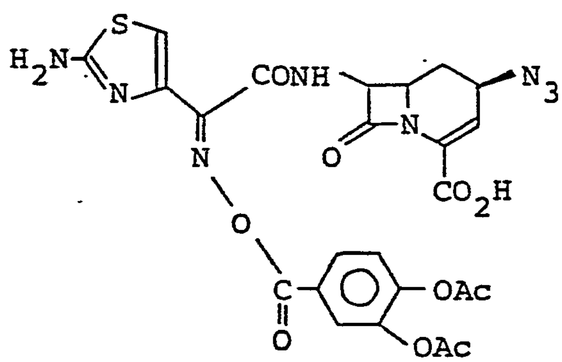

The same procedure as in Example 12 is repeated using 112 mg of [4R,6R,7S]-7-amino-4-azido-1-azabicyclo-[4,2,0]oct-2-en-8-oxo-2-carboxylic acid obtained according to the process of Example 41 in place of Compound a, whereby 70 mg of the desired compound is obtained as powder. Yield: 22.8%.

NMR($d_6$DMSO-$CD_3$OD) δ: 2.32(6H, s), 5.68(1H, d, J = 5.9Hz), 6.13(1H, d, J = 2.0Hz), 7.24(1H, s), 7.43 - 8.05 (3H, m)

IR $\nu_{max}^{cm^{-1}}$(KBr): 2110, 1790(sh), 1780, 1760, 1750(sh)

Example 7

Preparation of (±)-cis-7β-[2-(2-amino-4-thiazolyl)-2-syn-methoxyiminoacetamido]-4β-amino-1-azabicyclo[4,2,0]-oct-2-en-8-oxo-2-carboxylic acid hydrochloride:

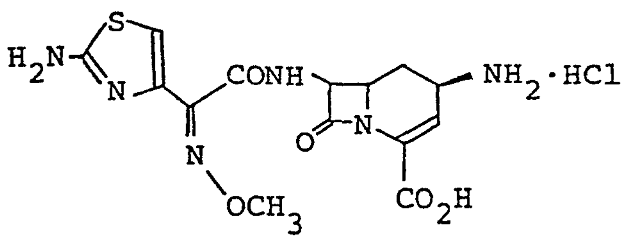

At first, 16 ml of aqueous 50% methanol, 0.6 ml of 1N hydrochloric acid and 56 mg of 5% palladium carbon catalyst are added to 113 mg of (±)-cis-7β-[2-(2-amino-4-thiazolyl)-2-syn-methoxyiminoacetamido]-4β-azido-1-azabicyclo[4,2,0]oct-2-en-8-oxo-2-carboxylic acid obtained according to the process of Example 3, and hydrogen is passed through the mixture at room temperature with stirring over 5 hours. The catalyst is removed by filtration, and the reaction mixture is concentrated to about 0.5 ml under reduced pressure and then purified with 15 ml of Diaion HP 20 AG resin. Fractions containing the desired compound, which are eluted with water, are concentrated under reduced pressure, whereby 68 mg of the desired compound is obtained as powder. Yield: 58.5%.

NMR($d_6$DMSO-$D_2$O) δ: 1.4 - 2.4(2H, m), 3.91(3H, s), 4.03(2H, m), 5.39(1H, d, J = 5.4Hz), 5.69(1H, d, J = 1.5Hz), 6.83(1H, s)

IR $\nu_{max}^{cm^{-1}}$ (KBr): 1755, 1750(sh), 1620 - 1670

Example 8

Preparation of (±)-cis-7β-[2-(2-amino-4-thiazolyl)-2-syn-methoxyiminoacetamido]-4β-acetamino-1-azabicyclo-[4,2,0]oct-2-en-8-oxo-2-carboxylic acid:

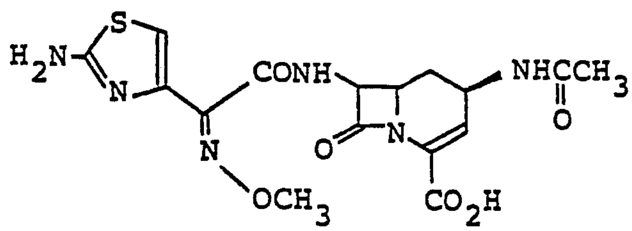

At first, 25 mg of (±)-cis-7β-[2-(2-amino-4-thiazolyl)-2-syn-methoxyiminoacetamido]-4β-amino-1-azabicyclo[4,2,0]oct-2-en-8-oxo-2-carboxylic acid hydrochloride is dissolved in 2 ml of anhydrous pyridine. Then, 30 μl of acetic anhydride is added thereto under ice cooling and with stirring, and the mixture is stirred at the same temperature for one hour. Ice is added to the reaction mixture, and the resulting mixture is stirred for 10 minutes and concentrated. The residue is dissolved in a small amount of dimethylsulfoxide, and the solution is purified with 10 ml of Diaion HP 20 AG resin. Fractions containing the desired compound, which are eluted with water-methanol (4 : 1), are concentrated under reduced pressure to obtain 14 mg of the desired compound as powder. Yield: 55.2%.

NMR($CD_3OD$-$D_2O$) δ: 1.32 - 2.42(2H, m), 2.02(3H, s), 3.97(3H, s), 4.21(1H, m), 5.45(1H, d, J = 5.2Hz), 6.04(1H, d, J = 2.4Hz), 6.91(1H, s)

IR $\nu_{max}^{cm^{-1}}$ (KBr): 1780(sh), 1770, 1760(sh), 1620 - 1680

Example 9

Preparation of (±)-cis-7β-[2-(2-amino-4-thiazolyl)-2-syn-methoxyiminoacetamido]-4β-N-methylureido-1-azabicyclo[4,2,0]oct-2-en-8-oxo-2-carboxylic acid:

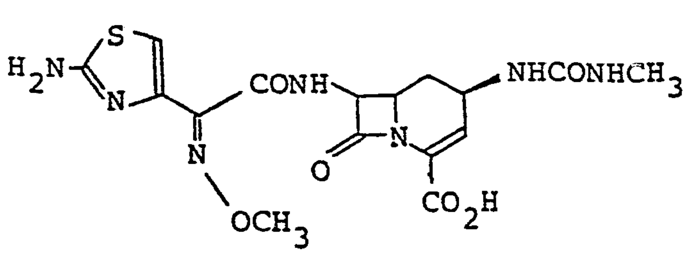

At first, 45 mg of (±)-cis-7β-[2-(2-amino-4-thiazolyl)-2-syn-methoxyiminoacetamido]-4β-amino-1-azabicyclo[4,2,0]oct-2-en-8-oxo-2-carboxylic acid hydrochloride, obtained according to the process of Reference Example 7 is dissolved in 3 ml of anhydrous dimethylformamide. Then, 70 μl of triethylamine is added thereto under ice cooling and with stirring, thereafter 9 μl of methyl isocyanate is added thereto, and the mixture is subjected to reaction at the same temperature for 45 minutes. Then, 3 ml of water is added to the reaction mixture, and the mixture is adjusted to pH 3.0 with 1N hydrochloric acid. The reaction mixture is concentrated to 0.5 ml under reduced pressure, and a small amount of water is added thereto to obtain a homogenous solution. The solution is subjected to purification with 20 ml of Diaion HP 20 AG resin. Fractions containing the desired compound, which are eluted with water-methanol (4 : 1), are concentrated under reduced pressure to obtain 40 mg of desired compound as powder. Yield: 84.7%.

NMR($d_6$DMSO-$D_2$O)δ: 2.63(3H, s), 3.90(3H, s), 4.57(1H, m), 5.42(1H, d, J = 5.2Hz), 6.12(1H, d, J = 2.0Hz), 6.83(1H, s)

IR $\nu_{max}^{cm^{-1}}$ (KBr): 1775, 1765, 1755, 1620 - 1680

Example 10

Preparation of (±)-cis-7β-[2-(2-amino-4-thiazolyl)-2-syn-methoxyiminoacetamido]-4β-ureido-1-azabicyclo[4,2,0]oct-2-en-8-oxo-2-carboxylic acid:

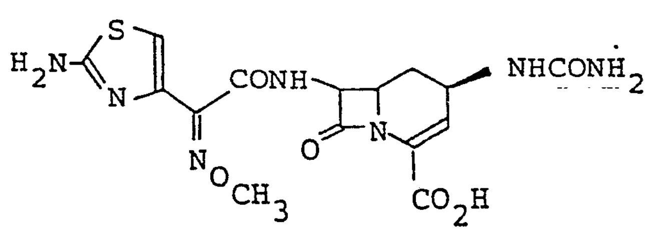

At first, 1.5 ml of dimethylformamide, 0.2 ml of acetic acid and 3 ml of water are added to 12 mg of (±)-cis-7β-[2-(2-amino-4-thiazolyl)-2-syn-methoxyimino-acetamido]-4β-amino-1-azabicyclo[4,2,0]oct-2-en-8-oxo-2-carboxylic acid hydrochloride obtained according to the process of Example 7, and then 20 mg of sodium cyanate is added to the mixture. The resulting mixture is stirred at room temperature for 2 hours. The reaction mixture is concentrated under reduced pressure, and purified with 10 ml of Diaion HP-20 AG resin. Fractions containing the desired compound, which are eluted with water-methanol (8 : 1), are concentrated under reduced pressure to obtain 10 mg of the desired compound as powder. Yield: 82.0%.

NMR($CD_3OD-D_2O$)δ: 3.97(3H, s), 5.43(1H, d, J = 5.1Hz), 6.05(1H, brs), 6.85(1H,s)

IR $\nu_{max}^{cm^{-1}}$ (KBr): 1770(sh), 1765, 1760(sh), 1630 - 1680

<u>Example 11</u>

Preparation of [4S,6R,7S]-7-[2-(2-amino-4-thiazolyl)-2-syn-methoxyiminoacetamido]-4-methoxy-1-azabicyclo[4,2,0]oct-2-en-8-oxo-2-carboxylic acid:

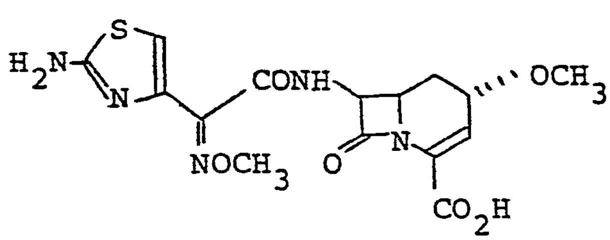

The same procedure as in Example 1 is repeated using 43.3 mg of [4S,6R,7S]-7-amino-4-methoxy-1-azabicyclo [4,2,0]oct-2-en-8-oxo-2-carboxylic acid obtained according to the process of Example 46, and purification is carried out with 10 ml of Diaion HP-20 AG resin. Fractions containing the desired compound eluted by water-methanol (3 : 2) are concentrated under reduced pressure to obtain 36 mg of the desired compound as powder. Yield: 44.7%.

NMR($CD_3OC-D_2O$)δ: 1.53 - 2.43(2H, m), 3.45(3H, s), 3.98(3H, s), 3.8 - 4.2(2H, m), 5.58(1H, d, J = 5.4Hz), 6.46(1H, d, J = 5.0Hz), 6.87(1H, s)

IR $\nu_{max}^{cm^{-1}}$ (KBr): 1770, 1760(sh), 1670, 1630

$[\alpha]_D^{20}$ = -57.2° (c=0.5, methanol)

Example 12

Preparation of diphenylmethyl ester of [4R,6R,7S]-7-[2-(2-tritylamino-4-thiazolyl)-2-syn-methoxyiminoacetamido]-4-(1-phenyl-1,2,3,4-tetrazol-5-yl)-thio-1-azabicyclo[4,2,0] oct-2-en-8-oxo-2-carboxylic acid:

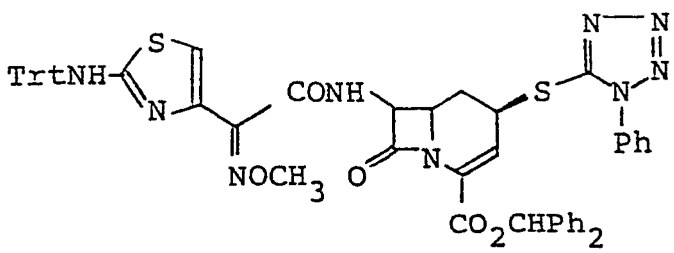

At first, 500 mg of diphenylmethyl ester of [4S,6R,7S]-7-[2-(2-tritylamino-4-thiazolyl)-2-syn-methoxyiminoacetamido]-4-hydroxy-1-azabicyclo[4,2,0]oct-2-en-8-oxo-2-carboxylic acid (hereinafter referred to as "Compound b") obtained according to the process of Reference Example 1, is dissolved in 5.7 ml of anhydrous dimethylformamide. Then, 333 mg of triphenylphosphine is added thereto under ice cooling and with stirring, and then 226 mg of N-bromosuccinimide is added thereto in 5 portions over 15 minutes. The mixture is stirred at the same temperature for one hour.

On the other hand, 169 mg of 1-phenyl-1,2,3,4-tetrazol-5-thiol is dissolved in 1.5 ml of dimethylformamide, and 45 mg of 50% sodium hydride is added thereto under ice cooling. The mixture is stirred as such at room temperature for 30 minutes. The resulting solution is added to the said reaction mixture, and then the mixture is stirred under ice cooling for one hour. Then, 0.5N hydrochlonic acid is added to the reaction mixture, and the mixture is extracted twice with ethyl acetate. The extract is washed three times with water and once with a saturated sodium chloride solution, and dried over sodium sulfate. The solvent is distilled off under reduced pressure, and the oily residue is subjected to purification by silica gel chromatography (50 g, n-hexane : ethyl acetate = 1 : 1), whereby 470 mg of the desired compound is obtained as powder. Yield: 78.5%.

NMR($CD_3OD$-$CDCl_3$)$\delta$: 3.90(3H, s), 5.50(1H, d, J = 5.0Hz), 6.50(1H, d, J = 2.0Hz), 6.67(1H, s), 6.93(1H, s), 7.27 - 7.63(30H, m)

IR $\nu_{max}^{cm^{-1}}$ ($CHCl_3$): 1790, 1740, 1690

Example 13

Preparation of [4R,6R,7S]-7-[2-(2-amino-4-thiazolyl)-2-syn-methoxyiminoacetamido]-4-(1-phenyl-1,2,3,4-tetrazol-5-yl)-thio-1-azabicyclo[4,2,0]oct-2-en-8-oxo-2-carboxylic acid:

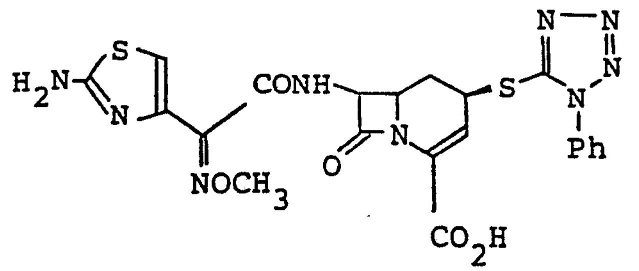

At first, 470 mg of diphenylmethyl ester of [4R,6R,7S]-7-[2-(2-tritylamino-4-thiazolyl)-2-syn-methoxyiminoacetamido]-4-(1-phenyl-1,2,3,4-tetrazol-5-yl)-thio-1-azabicyclo[4,2,0]oct-2-en-8-oxo-2-carboxylic acid (hereinafter referred to as "Compound c") obtained according to the process of Example 12, is dissolved in 30 ml of anhydrous methylene chloride, and 7.5 ml of trifluoroacetic acid is added thereto under ice cooling. The mixture is allowed to stand at the same temperature for 30 minutes. The reaction mixture is concentrated under reduced pressure, and ethyl acetate is added to the residue, and the mixture is again concentrated under reduced pressure. This operation is repeated three times.

Then, 20 ml of aqueous 50% acetic acid is added to the residue, and the mixture is stirred at 55°C for 45 minutes. The reaction mixture is concentrated under reduced pressure, the residue is dissolved in a small amount of dimethylsulfoxide, and the solution is subjected to adsorption on 60 ml of Diaion HP 10 resin in a column. Fractions containing the desired compound, which are eluted with water-methanol (1 : 2), are concentrated under reduced pressure. The resulting powder is disintegrated in a solution consisting of 25 ml of ethyl acetate and 75 ml of n-hexane, and the desired compound is separated by filtration, whereby 102 mg of the powder is obtained. Yield: 39.4%.

NMR($d_6$DMSO)δ: 1.51 - 2.47(2H, m), 3.75(3H, s), 4.07(1H, m), 4.84(1H, m), 5.52(1H, dd, J = 5.4Hz, 8.8Hz), 6.31(1H, d, J = 2.0Hz), 6.76(1H, s), 7.17(2H, brs), 7.66(5H, s), 9.26(1H, d, J = 8.8Hz)

IR $\nu_{max}^{cm^{-1}}$ (KBr): 1790(sh), 1775, 1770(sh), 1660, 1630

Example 14

Preparation of diphenylmethyl ester of [4R,6R,7S]-7-[2-(2-tritylamino-4-thiazolyl)-2-syn-methoxyiminoacetamido]-4-(1-methyl-1,2,3,4-tetrazol-5-yl)-thio-1-azabicyclo[4,2,0] oct-2-en-oxo-2-carboxylic acid:

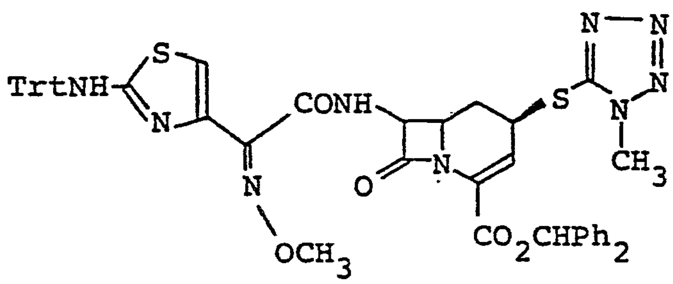

The same procedure as in Example 12 is repeated using 1.19 g of diphenylmethyl ester of [4S,6R,7S]-7-[2-(2-tritylamino-4-thiazolyl)-2-syn-methoxyiminoacetamido]-4-hydroxy-1-azabicyclo[4,2,0]oct-2-en-8-oxo-2-carboxylic acid obtained according to the process of Reference Example 1 in place of compound 6 and 348 mg of 1-methyl-1,2,3,4-tetrazol-5-thiol, whereby 722 mg of the desired compound is obtained as powder. Yield: 58.0%.

NMR($CDCl_3$-$CD_3OD$) $\delta$: 3.90(3H, s), 3.97(3H, s), 4.67 (1H, m), 5.50(1H, d, J=5.5Hz), 6.43(1H, d, J=2.0Hz), 6.67(1H, s), 6.93(1H, s), 7.33(25H, m)

IR $\nu_{max}^{cm^{-1}}$ ($CHCl_3$): 1795, 1790(sh), 1740, 1695, 1690

Example 15

Preparation of [4R,6R,7S]-7-[2-(2-amino-4-thiazolyl)-2-syn-methoxyiminoacetamido]-4-(1-methyl-1,2,3,4-tetrazol-5-yl)-thio-1-azabicyclo[4,2,0]oct-2-en-8-oxo-2-carboxylic acid:

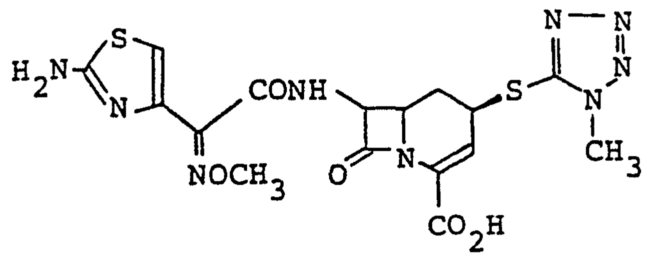

The same procedure as in Example 13 is repeated using 722 mg of diphenylmethyl ester of [4R,6R,7S]-7-[2-(2-tritylamino-4-thiazolyl)-2-syn-methoxyiminoacetamido]-4-(1-methyl-1,2,3,4-tetrazol-5-yl)-thio-1-azabicyclo[4,2,0]oct-2-en-8-oxo-2-carboxylic acid obtained according to the process of Example 15 in place of Compound c. Purification is carried out with 120 ml of Diaion HP 10 resin, and fractions containing the desired compound, which are eluted with water-methanol (1 : 1), are concentrated under reduced pressure to obtain 176 mg of the desired compound as powder. Yield: 42.2%.

NMR and IR data are identical with those of the corresponding dl isomer (Example 1).

Example 16

Preparation of diphenylmethyl ester of [4R,6R,7S]-7-[2-(2-tritylamino-4-thiazolyl)-2-syn-methoxyiminoacetamido]-4-azido-1-azabicyclo[4,2,0]oct-2-en-8-oxo-2-carboxylic acid:

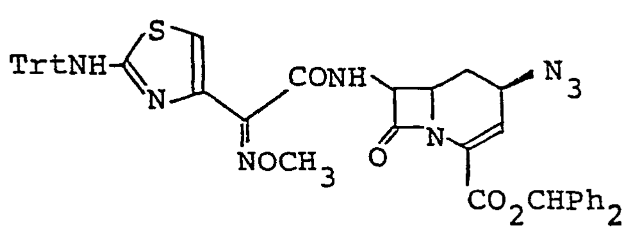

The same procedure as in Example 12 is repeated using 198 mg of diphenylmethyl ester of [4S,6R,7S]-7-[2-(2-tritylamino-4-thiazolyl)-2-syn-methoxyiminoacetamido]-4-hydroxy-1-azabicyclo[4,2,0]oct-2-en-8-oxo-2-carboxylic acid obtained according to the process of Reference Example 1

in place of Compound b and 65 mg of sodium azide. Purification is carried out according to silica gel chromatography (20 g, n-hexane : ethyl acetate = 2 : 1), whereby 119 mg of the desired compound is obtained as powder. Yield: 58.2%.

NMR($CDCl_3$-$CD_3OD$) δ: 1.50 - 2.57(2H, m), 4.00(3H, s), 5.42(1H, d, J = 5.5Hz), 6.32(1H, d, J = 2.0Hz), 6.63(1H, s), 6.93(1H, s), 7.33(25H, m)

IR $\nu_{max}^{cm^{-1}}$ ($CHCl_3$): 2100, 1795, 1785, 1740, 1695(sh), 1685

Example 17

Preparation of [4R,6R,7S]-7-[2-(2-amino-4-thiazolyl)-2-syn-methoxyiminoacetamido]-4-azido-1-azabicyclo[4,2,0]oct-2-en-8-oxo-2-carboxylic acid:

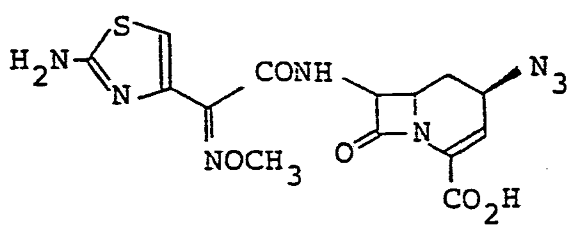

The same procedure as in Example 13 is repeated using 119 mg of diphenylmethyl ester of [4R,6R,7S]-7-[2-(2-tritylamino-4-thiazolyl)-2-syn-methoxyiminoacetamido]-4-azido-1-azabicyclo[4,2,0]oct-2-en-8-oxo-2-carboxylic acid obtained according to the process of Example 16 in place of Compound c. Purification is carried out with 20 ml of HP 10 resin, and fractions containing the desired compound, which are eluted with water-methanol (4 : 3), are concentrated under reduced pressure, whereby 26 mg of the desired compound is obtained as powder. Yield: 43.9%.

NMR and IR data are identical with those of the corresponding dl isomer (Example 3).

Example 18

Preparation of [4R,6R,7S]-7-[2-(2-amino-4-thiazolyl)-2-syn-methoxyiminoacetamido]-4-(1-carboxymethyl-1,2,3,4-tetrazol-5-yl)-thio-1-azabicyclo[4,2,0]oct-2-en-8-oxo-2-carboxylic acid:

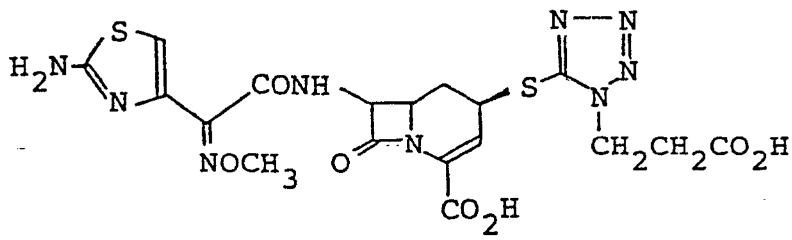

The same procedure as in Example 12 is repeated using 396 mg of diphenylmethyl ester of [4S,6R,7S]-7-[2-(2-tritylamino-4-thiazolyl)-2-syn-methoxyiminoacetamido]-4-hydroxy-1-azabicyclo[4,2,0]oct-2-en-8-oxo-2-carboxylic acid obtained according to the process of Reference Example 1 in place of Compound b and 160 mg of 1-carboxymethyl-1,2,3,4-tetrazole-5-thiol. However, the reaction product is subjected to removal of protective group without silica gel chromatographic purification, and then treated in the same manner as in Example 13. Purification is carried out with 60 ml of Diaion HP 10 resin, and fractions containing the desired compound, which are eluted with water-methanol (3 : 1), are concentrated under reduced pressure to obtain 80 mg of the desired compound as powder.

NMR($CD_3OD$-$D_2O$-$d_6DMSO$)$\delta$: 3.89(3H, s), 5.26(2H, s), 5.51(1H, d, J = 4.9Hz), 6.36(1H, d, J = 2.5Hz), 6.84(1H, s)

IR $\nu_{max}^{cm^{-1}}$ (KBr): 1785, 1780(sh), 1605 - 1695

Example 19

Preparation of sodium [4S,6R,7S]-7-[2-(2-aminothiazol-4-yl)-2-syn-methoxyiminoacetamido]-4-(5-methyl-1,3,4-thiadiazol-2-yl)-thio-1-azabicyclo[4,2,0]oct-2-en-8-oxo-2-carboxylate:

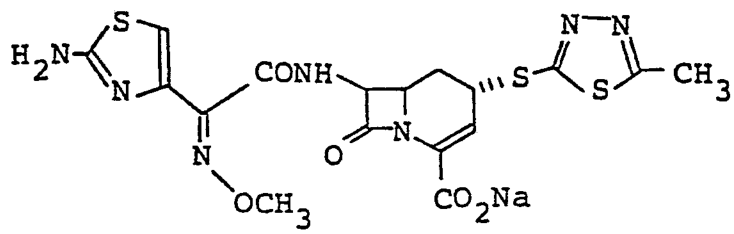

At first, 176 mg (0.416 m moles) of [4S,6R,7S]-7-[2-(2-aminothiazol-4-yl)-2-syn-methoxyiminoacetamido]-4-acetoxy-1-azabicyclo[4,2,0]oct-2-en-8-oxo-2-carboxylic acid obtained according to the process of Reference Example 4 (hereinafter referred to as "Compound e") is suspended in 8 ml of water, and the suspension is adjusted to pH 7.5 with a saturated sodium hydrogen carbonate solution and then admixed with water to make 10 ml of a solution. Then, 66 mg (0.499 m moles) of 5-methyl-2-mercapto-1,3,4-thiadiazole is added thereto and the mixture is stirred at 50°C for 2.5 hours. The mixture is allowed to stand at room temperature for 15 hours, and the reaction mixture is adjusted to pH 6.9 with dilute hydrochloric acid and concentrated under reduced pressure to about 5 ml. The concentrate is purified with 100 ml of Diaion HP 10 resin in a column. After washing with 200 ml of water and 200 ml of water-methanol (5 : 1), fractions eluted with water-methanol (1 : 2) are joined together, and concentrated under reduced pressure to obtain 71.6 mg (0.138 m moles) of the desired compound as yellow powder. Yield: 33.2%.

NMR($D_2O$-$CD_3OD$)δ: 6.91(1H, s), 6.20(1H, d, J = 5.4Hz), 5.62(1H, d, J = 5.4Hz), 3.95(3H, s), 2.76(3H, s), 2.6 - 1.9(2H, m)

IR $\nu_{max}^{cm^{-1}}$ (KBr): 3400, 1775(s), 1765, 1680(s), 1670

Example 20

Preparation of sodium [4S,6R,7S]-7-[2-(2-aminothiazol-4-yl)-2-syn-methoxyimino]acetamido-4-(2-amino-1,3,4-thiadiazol-5-yl)-thio-1-azabicyclo[4,2,0]oct-2-en-8-oxo-2-carboxylate:

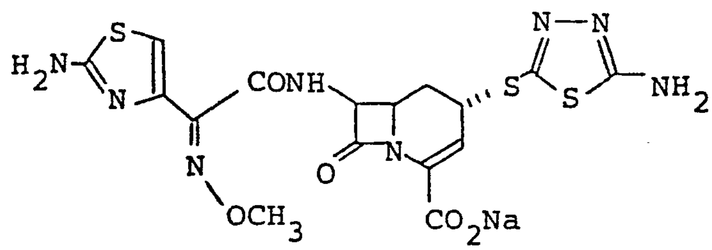

In this example, reaction of 176 mg (0.416 m moles) of Compound e with 66.5 mg (0.499 m moles) of 2-amino-5-mercapto-1,3,4-thiadiazole and purification are carried out in the same manner as in Example 19, and fractions eluted with water-methanol (5 : 1) are joined together and concentrated under reduced pressure to obtain 106.5 mg (0.205 m moles) of the desired compound as yellow powder. Yield: 41.1%.

NMR($D_2O$-$CD_3OD$)$\delta$: 6.88(1H, s), 6.15(1H, d, J = 5.4Hz), 5.62(1H, d, J = 4.9Hz), 4.6 - 3.9(2H, m), 3.94(3H, s), 2.4 - 1.8(2H, m)

IR $\nu_{max}^{cm^{-1}}$ (KBr): 3300, 1780(s), 1760, 1680(s), 1670

Example 21

Preparation of sodium [4S,6R,7S]-7-[2-(2-aminothiazol-4-yl)-2-syn-methoxyiminoacetamido]-4-(benzothiazol-2-yl)-thio-1-azabicyclo[4,2,0]oct-2-en-8-oxo-2-carboxylate:

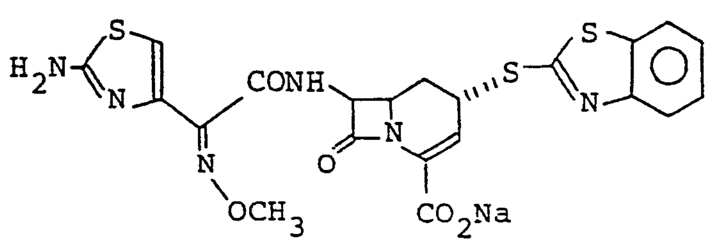

In this example, reaction of 134 mg (0.315 m moles) of Compound e with 105 mg (0.63 m moles) of 2-mercaptobenzothiazole and purification are carried out in the same manner as in Example 19, and fractions eluted with water-methanol (1 : 1 and 1 : 3) are joined together and concentrated

under reduced pressure to obtain 55.1 mg of yellow powder. The powder is purified with 10 ml of Diaion CHP-20P. After washing with 10 ml of water, 20 ml of water-methanol (8 : 1), 20 ml of water-methanol (5 : 1) and 10 ml of water-methanol (3 : 1), fractions eluted with water-methanol (1 : 1) are joined together and concentrated under reduced pressure to obtain 20.7 mg (0.0375 m moles) of the desired compound as yellow powder. Yield: 11.9%.

NMR($D_2O$-$CD_3OD$-DMSO-$d_6$)δ: 8.1 - 7.8(2H, m), 7.7 - 7.3 (2H, m), 6.82(1H, s), 6.13(1H, d, J = 5.4Hz), 5.60(1H, d, J = 4.9Hz), 5.1 - 4.9(1H, m), 4.3 - 4.0 (1H, m), 3.88(3H, s), 2.5 - 1.8(2H, m)

IR $\nu_{max}^{cm^{-1}}$ (KBr): 3400, 1780(s), 1765, 1680, 1660

Example 22

Preparation of sodium [4S,6R,7S]-7-[2-(2-aminothiazol-4-yl)-2-syn-methoxyimino]acetamido-4-(pyridin-2-yl)-thio-1-azabicyclo[4,2,0]oct-2-en-8-oxo-2-carboxylate:

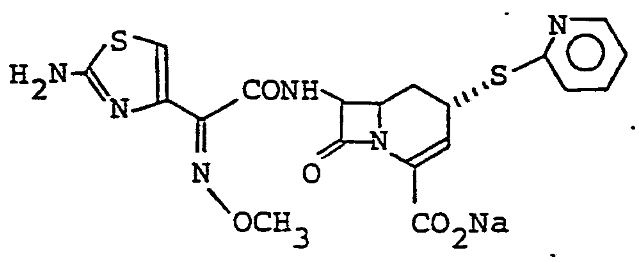

At first, 400 mg (0.945 m moles) of Compound e is reacted with 177 mg (1.59 m moles) of 2-mercaptopyridine in the same manner as in Example 19. The reaction mixture is purified with 180 ml of Diaion HP 10. After washing with 400 ml of water, 400 ml of water-methanol (6 : 1), 300 ml of water-methanol (3 : 1) and 100 ml of water-methanol (1 : 1), fractions eluted with water-methanol (1 : 1 and 1 : 3) are joined together and concentrated under reduced pressure. The solid matters are dissolved in 5 ml of methanol, and 50 ml of ether is added thereto. The resulting precipitate is separated by centrifugation and dried to obtain 70.6 mg

(0.142 m moles) of the desired compound as yellow powder. Yield: 15.0%.

NMR(DMSO-$d_6$) δ: 9.28 (1H, d, J = 8.8Hz), 8.46 (1H, d, J = 4.4Hz), 7.76 - 7.21 (2H, m), 6.73 (1H, s), 5.97 (1H, d, J = 5.4Hz), 5.48 (1H, dd, J = 8.8, 5.4Hz), 4.35 (1H, m), 3.77 (3H, s), 2.1 - 1.9 (2H, m)

IR $\nu_{max}^{cm^{-1}}$ (KBr): 3400, 1775, 1765, 1685, 1675, 1635

Example 23

Preparation of sodium [4S,6R,7S]-7-[2-(2-aminothiazol-4-yl)-2-syn-methoxyimino]-acetamido-4-(pyridin-4-yl)-thio-1-azabicyclo[4,2,0]oct-2-en-8-oxo-2-carboxylate:

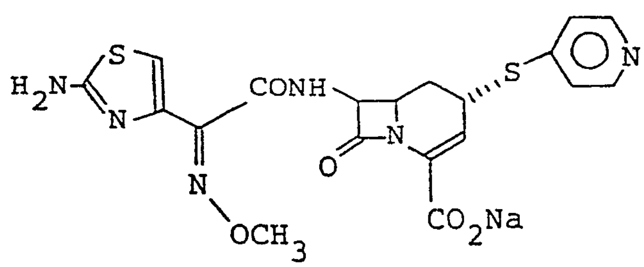

At first, 400 mg (0.945 m moles) of Compound e is reacted with 158 mg (1.42 m moles) of 4-mercaptopyridine in the same manner as in Example 19. The reaction mixture is washed three times with 10 ml of ethyl acetate, and adjusted to pH 6 with 6N hydrochloric acid. The mixture is concentrated under reduced pressure to about 5 ml, adjusted to pH 3 with 2N hydrochloric acid and purified with 200 ml of Diaion HP 10 resin. After washing with 500 ml of water, 550 ml of water-methanol (10 : 1), 600 ml of water-methanol (5 : 1), and 450 ml of water-methanol (2 : 1), fractions eluted with water-methanol (1 : 2) are joined together and concentrated under reduced pressure to obtain 250 mg of yellow powder. The powder is dissolved in an aqueous sodium hydrogen carbonate solution, and the solution is purified with 50 ml of Diaion HP 10 resin. After washing with 200 ml of water and 150 ml of water-methanol (5 : 1), fractions eluted with water-methanol (5 : 1 and 4 : 1) are

joined together and concentrated under reduced pressure to obtain 105 mg of yellow powder. The same purification is repeated, and fractions containing the desired compound are joined together and concentrated under reduced pressure to obtain 78.2 mg (0.157 m moles) of yellow powder. Yield: 16.6%.

NMR($D_2O$)δ: 8.40 - 8.23(2H, m), 7.44 - 7.31(2H, m), 6.88(1H, s), 6.17(1H, d, J = 5.4Hz), 5.59(1H, d, J = 5.1Hz), 4.7 - 4.6(1H, m), 4.3 - 4.1(1H, m), 3.91(3H, s), 2.2 - 1.9(2H, m)

IR $\nu_{max}^{cm^{-1}}$ (KBr): 3300, 1780(s), 1760, 1680, 1670

Example 24

Preparation of sodium [4S,6R,7S]-7-[2-(2-aminothiazol-4-yl)-2-syn-methoxyimino]-acetamido-4-(1H-1,2,4-triazol-3-yl)-thio-1-azabicyclo[4,2,0]oct-2-en-8-oxo-2-carboxylate:

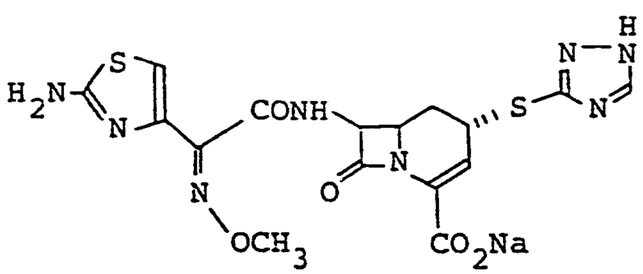

At first, 400 mg (0.945 m moles) of Compound e is reacted with 143 mg (1.42 m moles) of 1H-1,2,4-triazole-3-thiol at 50°C for 5 hours in the same manner as in Example 19. After cooling, the reaction mixture is purified with 200 ml of Diaion HP 10 resin. After washing with 500 ml of water, fractions eluted with water-methanol (8 : 1 and 5 : 1) are joined together, and concentrated under reduced pressure to obtain 219 mg of yellow powder. The powder is dissolved in an aqueous sodium hydrogen carbonate, and the solution is repurified with 50 ml of Diaion HP 10 resin. After washing with 100 ml of water, fractions eluted with water-methanol (10 : 1) are joined together, and concentrated

under reduced pressure to obtain 132 mg (0.271 m moles) of the desired compound as yellow powder. Yield: 28.7%.

NMR($D_2O$-$CD_3OD$)δ: 8.41(1H, s), 6.86(1H, s), 6.11(1H, d, J = 4.9Hz), 5.55(1H, d, J = 5.4Hz), 4.6 - 4.4 (1H, m), 4.3 - 4.0(1H, m), 3.86(3H, s), 2.3 - 1.9 (2H, m)

IR $\nu_{max}^{cm^{-1}}$ (KBr): 3300, 1760, 1675, 1600

Example 25

Preparation of [4S,6R,7S]-7-[2-(2-amino-4-thiazolyl)-2-syn-methoxyiminoacetamido]-4-(1-methyl-1,2,3,4-tetrazol-5-yl)-thio-1-azabicyclo[4,2,0]oct-2-en-8-oxo-2-carboxylic acid:

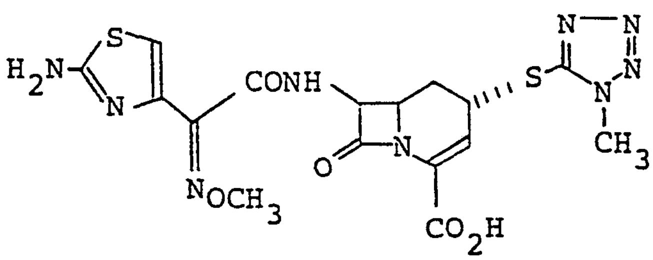

At first, 450 mg of Compound e is reacted with 248 mg of 1-methyl-1,2,3,4-tetrazole-5-thiol in the same manner as in Example 19. The reaction mixture is adjusted to pH 2.5 with 0.5N hydrochloric acid, and concentrated under reduced pressure. The residue is dissolved in dimethylsulfoxide, and the solution is purified with 120 ml of Diaion HP 10 resin. Fractions eluted with water-methanol (4 : 3) are joined together, and concentrated under reduced pressure to obtain 116 mg of the desired compound as powder. Yield: 22.8%.

NMR and IR data are identical with those of the corresponding dl isomer (Example 2).

Example 26

Preparation of [4S,6R,7S]-7-[2-(2-amino-4-thiazolyl)-2-syn-methoxyiminoacetamido]-4-azido-1-azabicyclo[4,2,0]oct-2-en-8-oxo-2-carboxylic acid:

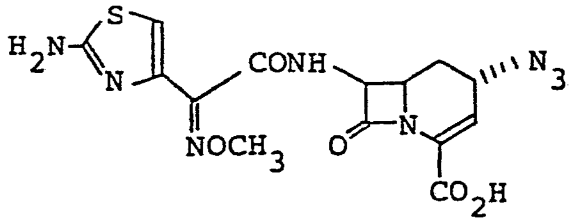

At first, 150 mg of Compound e is reacted with 46 mg of sodium azide in the same manner as in Example 19. The reaction mixture is adjusted to pH 2.5 with 0.5N hydrochloric acid, and concentrated under reduced pressure. The residue is dissolved in a small amount of dimethylsulfoxide, and the solution is purified with 20 ml of Diaion HP 10 resin. Fractions eluted with water-methanol (4 : 3) are joined together and concentrated under reduced pressure to obtain 44 mg of the desired compound as powder.
Yield: 30.6%.

NMR($CD_3OD$)δ: 1.5 - 2.4(2H, m), 3.97(3H, s), 4.47(1H, m), 5.60(1H, d, J = 5.4Hz), 6.43(1H, d, J = 5.4Hz), 6.83(1H, s)

IR $\nu_{max}^{cm^{-1}}$ (KBr): 2120, 1785(sh), 1775, 1620 - 1685

Example 27

Preparation of [4S,6R,7S]-7-[2-(2-aminothiazol-4-yl)-2-syn-methoxyimino]-acetamido-4-(pyridin-1-yl)-1-azabicyclo[4,2,0]oct-2-en-8-oxo-2-carboxylic acid:

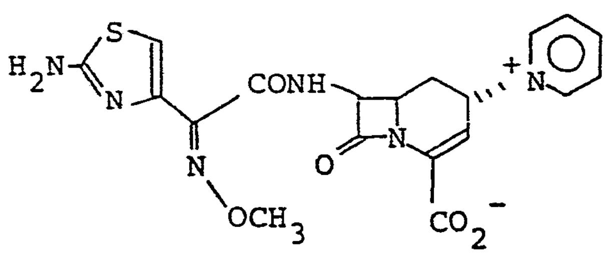

At first, 400 mg (0.944 m moles) of Compound e and 1.98 g (13.2 m moles) of sodium iodide are suspended in 0.5 ml of water and the suspension is heated at 80°C.

Then, 0.54 ml (6.72 m moles) of pyridine is added thereto, and the mixture is heared at 80°C with stirring for 50 minutes. After cooling to room temperature, the mixture is diluted with 100 ml of water, and concentrated under reduced pressure to about 50 ml to distill off pyridine. Then, 50 ml of water is added to the concentrate, and two drops of methyl isobutyl ketone are added thereto. The mixture is adjusted to pH 1.0 with 2N hydrochloric acid, washed twice with 40 ml of ethyl acetate, adjusted to pH 6.0 with an aqueous 1N sodium hydroxide solution and then concentrated under reduced pressure. The residue is purified with 130 ml of ion exchange resin Amberlite XAD-2 (made by Rohm and Haas Co.). After washing with 200 ml of water, elution is carried out with 10% aqueous ethanol. Fractions containing the desired compound are joined together, and concentrated under reduced pressure to obtain 187 mg (0.40 m moles) of the desired compound as yellow powder. Yield: 42.5%.

NMR($D_2O$)δ: 9.1 - 7.9(5H, m), 6.94(1H, s), 6.14(1H, d, J = 5.4Hz), 5.9 - 5.8(1H, m), 5.62(1H, d, J = 5.1Hz), 4.01(3H, s), 4.1 - 3.9(1H, m), 2.6 - 2.3(2H, m)

IR $\nu_{max}^{cm^{-1}}$ (KBr): 3400, 1775(s), 1765, 1750, 1665, 1630, 1624, 1540

Example 28

Synthesis of sodium salt of (4R,6R,7S)-7-[2-(2-aminothiazol-4-yl)-2-syn-[1-(3,4-dihydroxybenzoyl)-1-methyl-1-ethyl] oxyiminoacetamido-4-azido-1-azabicyclo[4,2,0]oct-2-en-8-oxo-2-carboxylic acid:

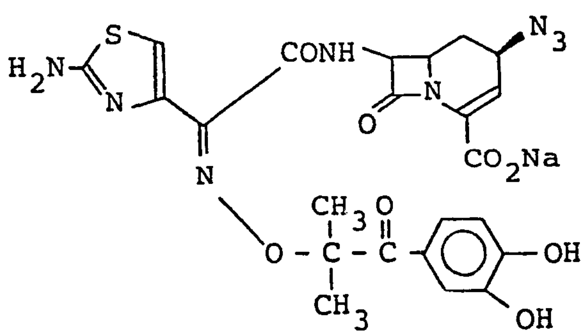

At first, 706 mg of 2-(2-tritylaminothiazol-4-yl)-2-syn-[1-(3,4-diacetoxybenzoyl)-1-methyl-1-ethyl] oxyiminoacetic acid is dissolved in 9.3 ml of anhydrous tetrahydrofuran, and 143 µl of triethylamine is added thereto. The mixture is cooled to -15°C with stirring. Then, 213 mg of phosphorus pentachloride is added thereto, and the mixture is stirred for 30 minutes.

On the other hand, 207 mg of (4R,6R,7S)-7-amino-4-azido-1-azabicyclo[4,2,0]oct-2-en-8-oxo-2-carboxylic acid is suspended in 9.3 ml of water and 9.3 ml of tetrahydrofuran, and the mixture is adjusted to pH 7.5 with triethylamine. Then, the said acid chloride solution is added dropwise to the mixture under ice cooling over 15 minutes, while keeping the mixture at pH 7 - 7.5 with triethylamine. Then, the mixture is stirred under ice cooling for 45 minutes. Then, 1N hydrochloric acid is added to the reaction mixture to make pH 1.5. Then, 50 ml of water is added thereto, and the mixture is extracted twice with 100 ml of ethyl acetate. The ethyl acetate layer is washed with a saturated aqueous sodium chloride solution, dried over sodium sulfate and concentrated under reduced pressure to obtain 830 mg of yellow solid. Then, 20 ml of acetic acid is added to the yellow solid, and also 20 ml of water is added dropwise thereto with heating to 50 - 55°C and stirring. Then, the mixture is stirred at the same temperature for 30 minutes, and then the reaction mixture is cooled. The precipitated crystals are removed by filtration, and the filtrate is concentrated under reduced pressure. The residue is dissolved in 40 ml of methanol and 20 ml of water, and a saturated aqueous sodium hydrogen carbonate solution is added thereto. The mixture is stirred at room temperature for 4 hours while keeping the mixture at pH 7.0. Then, the reaction mixture is adjusted to pH 7.0 with 1N hydrochloric acid, and concentrated to about 10 ml under reduced pressure. The solution as such and the resulting solid matters, after dissolution in 2 ml of dimethylsulfoxide, are both adsorbed on 180 ml of Diaion HP 10 in a column, and the column is washed with 600 ml of water and then with

400 ml of water : methanol (10 : 1). Fractions containing the desired compound eluted with water : methanol (3 : 2) are collected and concentrated under reduced pressure to obtain 145 mg of the desired compound as pale brown solid matters. Yield: 26.4%.

IR $\nu_{max}^{cm^{-1}}$ (KBr): 2120, 1780(sh), 1775(sh), 1770, 1760(sh), 1670, 1600

NMR($D_2O$)δ: 7.50 - 7.73(2H, m), 6.68 - 7.00(1H, m), 6.72(1H, s), 5.94(1H, d, J = 1.7Hz), 5.39(1H, d, J = 4.9Hz), 3.94 - 4.54(2H, m), 1.46 - 2.46(2H, m), 1.58(brs, 6H)

Example 29

Synthesis of sodium salt of [4S,6R,7S]-7-[2-(2-aminothiazol-4-yl)-2-(3,4-dihydroxybenzamido)] acetamido-4-(pyridin-4-yl)-thio-1-azabicyclo[4,2,0]oct-2-en-8-oxo-2-carboxylic acid:

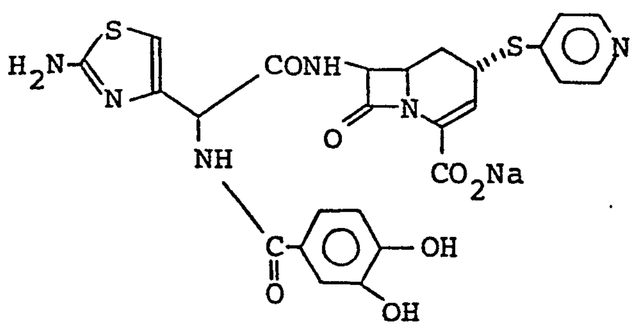

At first, 600 mg (1 m mole) of [4S,6R,7S]-7-[2-(2-aminothiazol-4-yl)-2-(3,4-diacetoxybenzamido)] acetamido-4-acetoxy-1-azabicyclo[4,2,0]oct-2-en-8-oxo-2-carboxylic acid, 167 mg (1.5 m moles) of 4-mercaptopyridine, and 336 mg (4 m moles) of sodium hydrogen carbonate are dissolved in 120 ml of water and the solution is stirred at 50°C for 3 hours. The reaction mixture is cooled to room temperature, and then subjected to column chromatography with 220 ml of Diaion HP-10. After washing with 1,000 ml of water and 500 ml of water : methanol (10 : 1), the fractions eluted with water : methanol (8 : 1 - 2 : 1) are collected and concentrated

under reduced pressure to obtain 340 mg of yellow solid matters. The solid matters are dissolved in water, and the solution is subjected to column chromatography with 50 ml of Diaion HP-10. After washing with 200 ml of water and 100 ml of water : methanol (8 : 1), the fractions eluted with water : methanol (2 : 1) are collected and concentrated under reduced pressure to obtain 180 mg (0.30 m moles) of the desired compound as yellow powder.

NMR($DMSO-D_2O$) δ ppm: 8.4 - 8.25(2H, m), 7.4 - 7.2(4H, m), 6.95(1H, d, J = 8Hz), 6.68(1H, s), 6.15 - 6.05 (1H, m), 5.53(1H, s), 5.38(1H, d, J = 4.6Hz), 4.7 - 4.3(1H, m), 4.2 - 3.8(1H, m), 2.2 - 1.5(2H, m)

IR (KBr) $\nu_{max}^{cm^{-1}}$ : 3350, 1760, 1610, 1585

Example 30

Synthesis of sodium salt of [4S,6R,7S]-7-[2-(2-aminothiazol-4-yl)-2-(3,4-dihydroxybenzamidoacetamido-4-(2-amino-1,3,4-thiadiazol-5-yl)-thio-1-azabicyclo[4,2,0]oct-2-en-8-oxo-2-carboxylic acid:

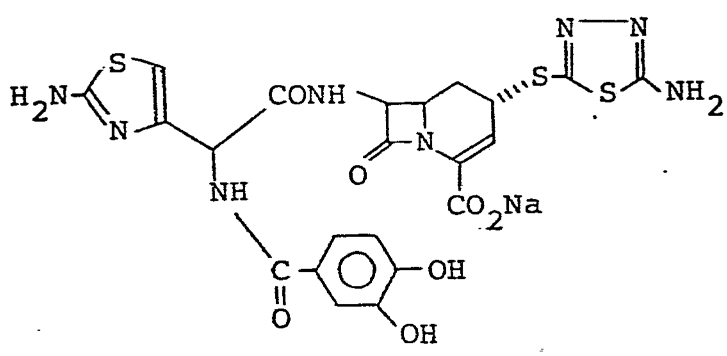

By reaction of 600 mg (1 m mole) of [4S,6R,7S]-7-[2-(2-aminothiazol-4-yl)-2-(3,4-diacetoxybenzamidoacetamido-4-acetoxy-1-azabicyclo[4,2,0]oct-2-en-8-oxo-2-carboxylic acid and 200 mg (1.5 m moles) of 2-amino-5-mercapto-1,3,4-thiadiazole and by successive purification in the same manner as in Example 29, 91 mg (0.145 m moles) of the desired compound is obtained as yellow powder.

NMR($D_2O$) δ ppm: 7.3 - 7.0(2H, m), 6.9 - 6.7(1H, m), 6.63 (1H, s), 6.08(1/2H, d, J = 5.4Hz), 6.07(1/2H, d,

J = 5.4Hz), 5.48(1H, s), 5.34(1H, d, J = 4.6Hz), 4.3 - 3.7(2H, m), 2.3 - 1.4(2H, m)

IR (KBr) $\nu_{max}^{cm^{-1}}$ : 3300, 1770, 1760, 1640, 1600, 1510

Example 31

Preparation of tertiary butyl ester of (±)-cis-7β-phthalimido-4β-(1-methyl-1,2,3,4-tetrazol-5-yl)-thio-1-azabicyclo[4,2,0]oct-2-en-8-oxo-2-carboxylic acid:

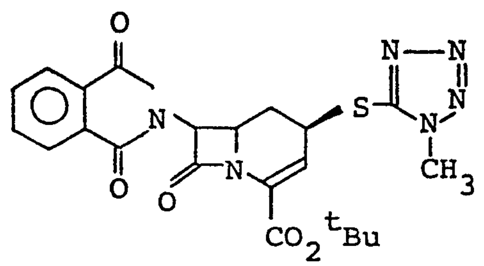

At first, 184 ml of anhydrous carbon tetrachloride, 1.78 g of N-bromosuccinimide and 0.53 g of azobisisobutyronitrile are added to 3.68 g of tertiary butyl ester of (±)-cis-4β-phthalimido-1-azabicyclo[4,2,0]oct-2-en-8-oxo-2-carboxylic acid, and the mixture is refluxed with heating and stirring for 15 minutes. After the solvent is distilled off, 20 ml of anhydrous dimethylformamide is added to the residue. While the resulting solution is cooled to 5°C with stirring, the following solution A is added thereto, and the mixture is stirred at room temperature for 45 minutes. [(preparation of solution A) 480 mg of 50% sodium hydride is added to a solution of 1.16 g of 1-methyl-1,2,3,4-tetrazol-5-thiol in 20 ml of anhydrous dimethylformamide at 0°C, and then the mixture is stirred at room temperature for 15 minutes]. Then, 1N hydrochloric acid is added to the reaction mixture to adjust pH to about 5, and then dimethylformamide is substantially distilled off. The residue is distributed in water-chloroform. The chloroform layer is washed four times with water and once with a saturated aqueous sodium chloride solution, and then dried over sodium sulfate. Then, the solution is concentrated under reduced pressure, and the residue is crystallized from ethyl acetate-n-hexane, whereby 2.71 g of the desired compound is obtained. Yield: 56.2%.

NMR($CDCl_3$)δ: 1.57(9H, s), 3.93(3H, s), 4.13(1H, m), 4.78(1H, m), 5.67(1H, d, J = 5.5Hz), 6.25(1H, d, J = 2.0Hz), 7.30(1H, s), 7.83(4H, m).

IR $\nu_{max}^{cm^{-1}}$ (KBr): 1805, 1795, 1785(sh), 1740, 1730(sh), 1630

Example 32

Preparation of tertiary butyl ester of (±)-cis-7β-amino-4β-(1-methyl-1,2,3,4-tetrazol-5-yl)-thio-1-azabicyclo[4,2,0]oct-2-en-8-oxo-2-carboxylic acid:

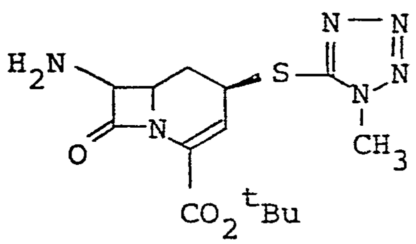

At first, 70 ml of tetrahydrofuran and 13 ml of water are added to 2.71 g of tertiary butyl ester of (±)-cis-7β-phthalimido-4β-(1-methyl-1,2,3,4-tetrazol-5-yl)-thio-1-azabicyclo[4,2,0]oct-2-en-8-oxo-2-carboxylic acid. The resulting solution is cooled to 0°C with stirring, and a solution of 1.88 g of sodium sulfide nonahydrate in 18 ml of water is added dropwise thereto over 8 minutes. The mixture is further stirred at the same temperature for 2 minutes, adjusted to about pH 6 with 1N hydrochloric acid, and concentrated under reduced pressure to distill off tetrahydrofuran. The solution is adjusted to pH 2 with 1N hydrochloric acid, and extracted twice with ethyl acetate. The organic layer is washed with a saturated aqueous sodium chloride solution, dried over sodium sulfate, and concentrated under reduced pressure to obtain crude oily matters. The oily matters are dissolved in 16 ml of anhydrous tetrahydrofuran, and 0.79 g of dicyclohexylcarbodiimide is added thereto at room temperature with stirring. The mixture is further stirred as it is for 45 minutes, and deposited urea

is removed by filtration. The filtrate is cooled to -78°C, and 190 μl of methylhydrazine is added dropwise thereto with stirring. The mixture is further stirred as it is for 20 minutes. Then, 4 ml of 1N hydrochloric acid is added to the reaction mixture to adjust pH to about 2, and the mixture is heated to room temperature over about 40 minutes. The mixture is further stirred at room temperature for 15 minutes, and concentrated under reduced pressure. Water is added to the residue, and precipitated insoluble matters are removed by filtration. The filtrate is washed twice with ethyl acetate, and the aqueous layer is adjusted to about pH 9 with a saturated aqueous sodium hydrogen carbonate under ice cooling and extracted 7 times with ethyl acetate. The organic layers are joined together, washed with a saturated aqueous sodium chloride solution, dried over sodium sulfate, and concentrated under reduced pressure to obtain 1.15 g of crude oily matters.

The oily matters are purified by silica gel chromatography (120 g, ethyl acetate-methanol = 30 : 1), whereby 347 mg of the desired compound is obtained. Yield: 17.5%.

In addition, 441 mg of tertiary butyl ester of (±)-cis-7β-amino-4α-(1-methyl-1,2,3,4-tetrazol-5-yl)-thio-1-azabicyclo[4,2,0]oct-2-en-8-oxo-2-carboxylic acid, which has undergone epimerization at position 4, is also obtained as a low polar compound (see Reference Example 7).

NMR($CDCl_3$)δ: 1.53(9H, s), 3.70 - 5.07(3H, m), 4.00(3H, s), 6.25(1H, brs)

IR $\nu_{max}^{cm^{-1}}$ ($CDCl_3$): 1790(sh), 1785, 1730, 1630

Example 33

Preparation of (±)-cis-7β-amino-4β-(1-methyl-1,2,3,4-tetrazol-5-yl)-thio-1-azabicyclo[4,2,0]oct-2-en-8-oxo-2-carboxylic acid trifluoroacetate:

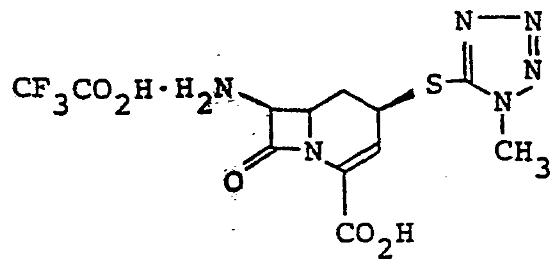

At first, 133 mg of tertiary butyl ester of (±)-cis-7β-amino-4β-(1-methyl-1,2,3,4-tetrazol-5-yl)-thio-1-azabicyclo[4,2,0]oct-2-en-8-oxo-2-carboxylic acid is dissolved in 2 ml of anhydrous methylene chloride, and 2 ml of trifluoroacetic acid is added thereto. The mixture is allowed to stand at room temperature for 90 minutes, and then concentrated under reduced pressure. Ethyl acetate is added to the residue, and the mixture is again concentrated under reduced pressure. This operation is repeated three times. The residue is disintegrated in 3 ml of ether-ethyl acetate (3 : 1) and subjected to filtration, whereby 123 mg of the desired compound is obtained as powder. Yield: 79.4%.

NMR($d_6$DMSO)δ: 3.99(3H, s), 4.57(2H, m), 6.18(1H, d, J = 2.5Hz)

IR $\nu_{max}^{cm^{-1}}$ (KBr): 1810(sh), 1805, 1620, 1550

Example 34

Preparation of tertiary butyl ester of (±)-cis-7β-phthalimido-4α-(1-methyl-1,2,3,4-tetrazol-5-yl)-thio-1-azabicyclo[4,2,0]oct-2-en-8-oxo-2-carboxylic acid:

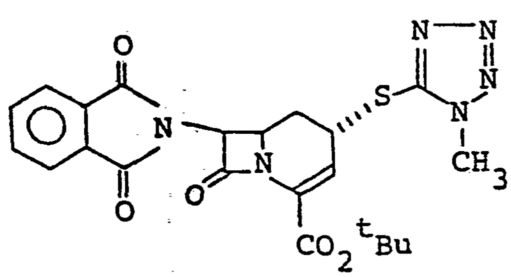

At first, 18 ml of anhydrous carbon tetrachloride, 178 mg of N-bromosuccinimide and 53 mg of azobisisobutyronitrile are added to 368 mg of tertiary butyl ester of (±)-cis-7β-phthalimido-1-azabicyclo[4,2,0]oct-2-en-8-oxo-2-carboxylic acid, and the mixture is refluxed with heating and stirring for 20 minutes. After the solvent is distilled off under reduced pressure, 5 ml of anhydrous methylene chloride is added to the residue, and then 116 mg of 1-methyl-1,2,3,4-tetrazol-5-thiol and 140 mg of zinc chloride are added thereto. The mixture is stirred at room temperature for 3 hours. The solvent is distilled off under reduced pressure, and the residue is purified by silica gel chromatography (30 g, chloroform - acetone = 30 : 1), whereby 156 mg of the desired compound is obtained. Yield: 32.3%.

NMR($CDCl_3$)δ: 1.57(9H, s), 3.90(3H, s), 4.15(1H, m), 4.83(1H, m), 5.73(1H, d, J = 5.7Hz), 6.32(1H, d, J = 5.7Hz), 7.25(1H, s), 7.83(4H, m)

IR $\nu_{max}^{cm^{-1}}$ (KBr): 1805(sh), 1800, 1790(sh), 1785, 1735, 1730, 1635

Example 35

Preparation of tertiary butyl ester of (±)-cis-7β-amino-4α-(1-methyl-1,2,3,4-tetrazol-5-yl)-thio-1-azabicyclo[4,2,0]oct-2-en-8-oxo-2-carboxylic acid:

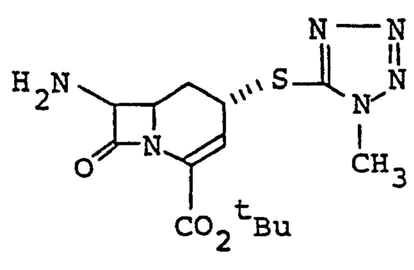

Dephthalylization is carried out in the same manner as in Example 32 with 813 mg of tertiary butyl ester of (±)-cis-7β-phthalimido-4α-(1-methyl-1,2,3,4-tetrazol-5-yl)-thio-1-azabicyclo[4,2,0]oct-2-en-8-oxo-2-carboxylic acid, whereby 193 mg of the desired compound is obtained. Yield: 32.4%.

The compound can also be prepared by epimerization according to the process of Reference Example 4.

NMR($CDCl_3$)δ: 1.57(9H, s), 3.80 - 5.07(3H, m), 3.97 (3H, s), 6.33(1H, d, J = 5.5Hz)

IR $\nu_{max}^{cm^{-1}}$ ($CDCl_3$): 1790(sh), 1780, 1730, 1630

Example 36

Preparation of (±)-cis-7β-amino-4α-(1-methyl-1,2,3,4-tetrazol-5-yl)-thio-1-azabicyclo[4,2,0]oct-2-en-8-oxo-2-carboxylic acid:

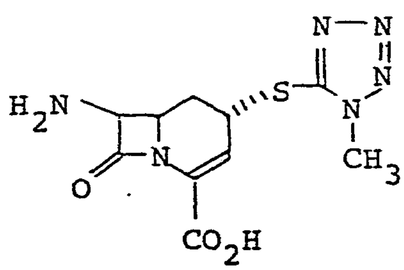

At first, 331 mg of tertiary butyl ester of (±)-cis-7β-amino-4α-(1-methyl-1,2,3,4-tetrazol-5-yl)-thio-1-azabicyclo[4,2,0]oct-2-en-8-oxo-2-carboxylic acid is dissolved in 4 ml of anhydrous methylene chloride, and 4 ml of trifluoroacetic acid is added thereto. The mixture is allowed to stand at room temperature for 90 minutes, and concentrated under reduced pressure. Ethyl acetate is added to the residue, and the mixture is again concentrated under reduced pressure. This operation is repeated three times.

Then, 10 ml of water and 3 ml of methanol are added to the residue, the mixture is adjusted to pH 3.5 with a saturated sodium hydrogen carbonate solution, and the resulting precipitate is separated by filtration. The precipitate is disintegrated in ethyl acetate, whereby 119 mg of the desired compound is obtained as powder. Yield: 42.7%.

NMR($d_6$DMSO)δ: 3.98(3H, s), 4.58(2H, m), 6.31(1H, d, J = 5.4Hz)

IR $\nu_{max}^{cm^{-1}}$(KBr): 1805(sh), 1800, 1630

Example 37

Preparation of tertiary butyl ester of (±)-cis-7β-phthalimido-4β-azido-1-azabicyclo[4,2,0]oct-2-en-8-oxo-2-carboxylic acid:

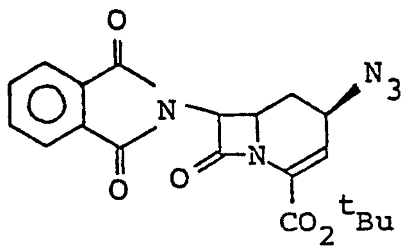

Reaction and extraction are carried out in the same manner as in Example 31 with 3.68 g of tertiary butyl ester of (±)-cis-7β-phthalimido-1-azabicyclo[4,2,0]oct-2-en-8-oxo-2-carboxylic acid and 780 mg of sodium azide. The organic layer is concentrated under reduced pressure, and the residue is purified by silica gel chromatography (150 g, n-hexane-ethyl acetate-chloroform = 3 : 2 : 1), whereby 3.0 g of the desired compound is obtained as powder. Yield: 74.0%.

NMR($CDCl_3$)δ: 1.57(9H, s), 3.93 - 4.47(2H, m), 5.57 (1H, d, J = 5.5Hz), 6.13(1H, d, J = 2.0Hz), 7.27 (1H, s), 7.85(4H, m)

IR $\nu_{max}^{cm^{-1}}$($CDCl_3$): 2100, 1800, 1790, 1730, 1630

Example 38

Preparation of tertiary butyl ester of (±)-cis-7β-amino-4β-azido-1-azabicyclo[4,2,0]oct-2-en-8-oxo-2-carboxylic acid:

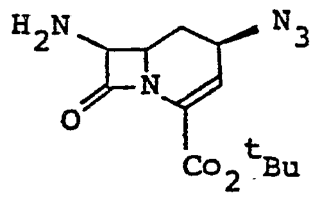

Reaction and extraction are carried out in the same manner as in Example 32 with 20 g of tertiary butyl ester of (±)-cis-7β-phthalimido-4β-azido-1-azabicyclo [4,2,0]oct-2-en-8-oxo-2-carboxylic acid, whereby 560 mg of the desired compound is obtained as oily matters. The oily matters are used in the next step without silica gel chromatographic purification. Yield: 41.0%.

NMR($CDCl_3$) δ: 1.57(9H, s), 3.77 - 4.60(3H, m), 6.08 (1H, d, J = 2.5Hz)

IR $\nu_{max}^{cm^{-1}}$ ($CHCl_3$): 2100, 1780(sh), 1775, 1720, 1630

Example 39

Preparation of (±)-cis-7β-amino-4β-azido-1-azabicyclo[4,2,0]oct-2-en-8-oxo-2-carboxylic acid trifluoroacetate:

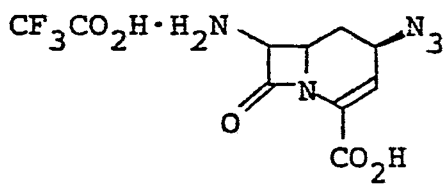

Reaction and purification are carried out in the same manner as in Example 33 with 560 mg of tertiary butyl ester of (±)-cis-7β-amino-4β-azido-1-azabicyclo[4,2,0] oct-2-en-8-oxo-2-carboxylic acid, whereby 336 mg of the desired compound is obtained as powder. Yield: 49.7%.

NMR($d_6$DMSO) δ: 3.99(3H, s), 4.57(2H, m), 6.18(1H, d, J = 2.5Hz)

IR $\nu_{max}^{cm^{-1}}$ (KBr): 1810(sh), 1805, 1620, 1550

Example 40

Preparation of sodium (±)-cis-7β-phenylacetamido-4β-azido-1-azabicyclo[4,2,0]oct-2-en-8-oxo-2-carboxylate:

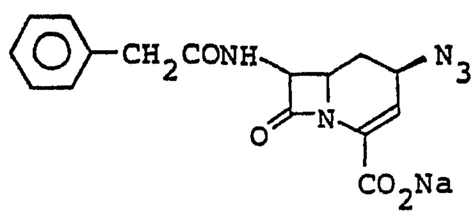

At first, 413 mg of (±)-cis-7β-amino-4β-azido-1-azabicyclo[4,2,0]oct-2-en-8-oxo-2-carboxylic acid trifluoroacetate is dissolved in 40 ml of aqueous 50% tetrahydrofuran, and sodium hydrogen carbonate powder is added thereto under ice cooling and stirring to adjust the pH of the solution to 7.5. A solution consisting of 1.1 ml of phenylacetyl chloride and 5 ml of tetrahydrofuran is added dropwise to the mixture at the same temperature, while keeping the pH to 7.2 - 7.5 with aqueous 50% sodium hydrogen carbonate during the reaction. The mixture is further stirred at the same temperature for one hour, and then tetrahydrofuran is distilled off under reduced pressure. The resulting aqueous solution is washed twice with ethyl acetate, and the aqueous layer is adjusted to pH 1.5 with 1N hydrochloric acid and extracted three times with ethyl acetate. After the solvent is distilled off under reduced pressure, the residue is dissolved in aqueous 50% methanol, and the solution is adjusted to pH 7.8 with a saturated aqueous sodium hydrogen carbonate solution. Then, methanol is distilled off under reduced pressure. The resulting aqueous solution is passed through a column of 40 ml of Diaion HP 20 AG resin, and the column is washed with 100 ml of water. Fractions containing the desired compound, which are eluted with water-methanol (8 : 1), are joined together and concentrated under reduced pressure to obtain 272 mg of the desired compound as powder. Yield: 61.1%.

NMR($CD_3OD$-$CDCl_3$)δ: 1.67(1H, m), 2.07(1H, m), 3.57 (2H, s), 3.97(1H, m), 4.27(1H, m), 5.23(1H, d, J = 5.0Hz), 5.97(1H, d, J = 2.0Hz), 7.27(5H, s)

IR $\nu_{max}^{cm^{-1}}$ (KBr): 2110, 1785, 1775, 1765, 1670, 1650, 1605

Example 41

Preparation of [4R,6R,7S]-7-amino-4-azido-1-azabicyclo[4,2,0]oct-2-en-8-oxo-2-carboxylic acid:

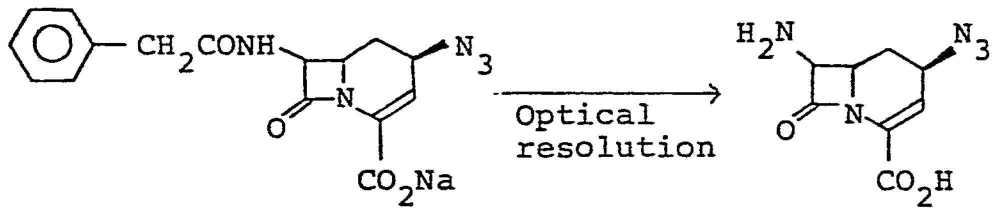


41-1) Preparation of cell fracture liquor

(a) Culturing of microorganism having an optically selective, deacylating property:

As a seed microorganism, Kluyvera citrophila ATCC 21285 is used [whose bacteriological properties are disclosed in J. General Applied Microbiology 3, 28 - 31 (1957)].

An aqueous solution containing 1% polypeptone, 1% yeast extract, 0.5% meat extract, 0.5% sodium glutamate and 0.25% sodium chloride, adjusted to pH 7.0 with 5N NaOH is used as a seed medium. One loopful of the seed microorganism is inoculated in 10 ml of the seed medium in a 50 ml-large test tube, and cultured with shaking at 30°C for 24 hours. The total amount of the seed medium is inoculated in 300 ml of a fermentation medium in a 2 ℓ-ribbed Erlenmeyer flask and culturing is carried out with shaking at 30°C.

The fermentation medium has the same composition as that of the seed medium.

(b) Preparation of cell fracture liquor

Cells are recovered from the fermentation liquor by centrifugation after 24 hours of the fermentation culturing, and washed twice with 50 ml of an aqueous 0.9% sodium chloride solution. The cells are suspended in a 1/30 M phosphate buffer. The cell concentration is 40 mg/ml, where the cell weight is based on dried cells. Then, 10 ml of the suspension is placed in a 50 ml-large test tube and subjected to ultrasonic treatment with 200 watts to fracture the cells, whereby a cell fracture liquor is prepared. The ultrasonic treatment is carried out by an ultrasonic generator Model UR200P, made by Tomy Seiko Co., Ltd., Japan.

41-2) Preparation of substrate solution

At first, 270 mg of sodium (±)-7β-phenylacetamido-4β-azido-1-azabicyclo[4,2,0]oct-2-en-8-oxo-2-carboxylate is added to 9 ml of a 1/30 M phosphate buffer (pH 6.5). The compound fails to dissolve at that time, but dissolves by adding thereto 2N NaOH by small portions and readjusting the pH to 6.5. Ultimately, deionized water is added thereto to make the volume 10 ml.

41-3) Enzyme reaction

At first, 10 ml of said cell fracture liquor is added to 10 ml of the substrate solution, and then, enzyme reaction is carried out at 30°C for 80 minutes.

41-4) Isolation and purification

After the end of the reaction, cells are removed from the reaction mixture by centrifugation, and the supernatant is adjusted to pH 3.0 with 1N hydrochloric acid. The resulting crystals of the desired compound are separated by filtration (27 mg). The filtrate is charged onto a column filled with 200 ml of Diaion HP 10 resin, and fractions containing the desired compound, which are eluted with water, are joined together, concentrated under reduced pressure and freeze-dried to obtain 26 mg of white powder. By joining together, 53 mg of the desired compound is obtained. Yield: 63.9%.

$[\alpha]_D^{22} = +141.8°$ (c=0.65, 50% aqueous tetrahydrofuran)

* adjusted to pH 8.0 with triethylamine

NMR($d_6$DMSO-$D_2$O)δ: 1.51 - 2.43(2H, m), 3.83(1H, m), 4.41(1H, d, J = 5.9Hz), 4.50(1H, m), 5.98(1H, d, J = 2.0Hz)

IR $\nu_{max}^{cm^{-1}}$ (KBr): 2200, 1800, 1790(sh), 1780(sh), 1620, 1550

Example 42

Preparation of tertiary butyl ester of (±)-cis-7β-phthalimido-4α-methoxy-1-azabicyclo[4,2,0]oct-2-en-8-oxo-2-carboxylic acid:

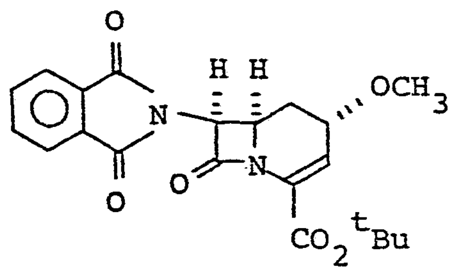

At first, 62 ml of anhydrous carbon tetrachloride, 0.59 g of N-bromosuccinimide and 0.19 g of azobisisobutyronitrile are added to 1.23 g of tertiary butyl ester of (±)-cis-7β-phthalimido-1-azabicyclo[4,2,0]oct-2-en-8-oxo-2-carboxylic acid, and the mixture is refluxed with heating and stirring for 40 minutes. Then, the reaction mixture is cooled to 70°C, and 10 ml of anhydrous methanol is added thereto. The mixture is refluxed with heating for 20 minutes. The reaction mixture is concentrated under reduced pressure, and 5 ml of ethyl acetate, 10 ml of ether and 15 ml of n-hexane are added to the residue. The deposited crystals are separated by filtration, whereby 0.87 g of the desired compound is obtained. Yield: 65.5%.

Physical properties of the compound are given below:

IR $\nu_{max}^{cm^{-1}}$ (KBr): 1800(sh), 1795(sh), 1780, 1735, 1725(sh)

NMR($CDCl_3$)δ: 7.82(4H, m), 6.40(1H, d, J = 5Hz), 5.70(1H, d, J = 6Hz), 3.87 - 4.20(2H, m), 3.37 (3H, s), 1.1 - 2.2(2H, m), 1.57(9H, s)

Example 43

Preparation of (±)-cis-7β-phthalimido-4α-methoxy-1-azabicyclo[4,2,0]oct-2-en-8-oxo-2-carboxylic acid:

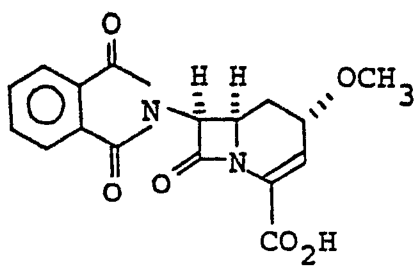

At first, 150 mg of tertiary butyl ester of (±)-cis-7β-phthalimido-4α-methoxy-1-azabicyclo[4,2,0]oct-2-en-8-oxo-2-carboxylic acid is dissolved in 2 ml of anhydrous methylene chloride, and 2 ml of trifluoroacetic acid is added thereto. The mixture is allowed to stand at room temperature for one hour. The reaction mixture is concentrated under reduced pressure, ethyl acetate is added thereto, and the mixture is re-concentrated under reduced pressure. The residue is washed with ether, whereby 119 mg of the desired compound is obtained as white powder. Yield: 92.0%.

IR $\nu_{max}^{cm^{-1}}$ (KBr): 1805(sh), 1800, 1790, 1730(sh), 1725

NMR($CDCl_3$-$CD_3OD$)δ: 7.85(4H, m), 6.55(1H, d, J = 5Hz), 5.75(1H, d, J = 5.5Hz), 3.9 - 4.3(2H, m), 3.40(3H, s), 1.2 - 2.3(2H, m)

Example 44

Preparation of (±)-cis-7β-amino-4α-methoxy-1-azabicyclo[4,2,0]oct-2-en-8-oxo-2-carboxylic acid:

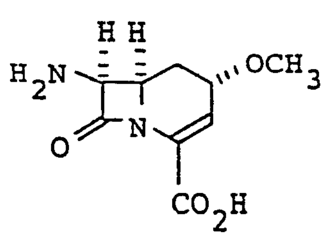

At first, 119 mg of (±)-cis-7β-phthalimido-4α-methoxy-1-azabicyclo[4,2,0]oct-2-en-8-oxo-2-carboxylic acid is dissolved in 1.9 ml of an aqueous 0.2N sodium hydrogen carbonate solution, and a solution consisting of 30 μl of hydrazine hydrate and 270 μl of water is added thereto under ice cooling over 15 minutes. The mixture as such is subjected to reaction for one hour.

The reaction mixture is adjusted to pH 1.5 with 1N hydrochloric acid, and the mixture is stirred at room temperature for two hours. The deposited crystals are removed by filtration, and the filtrate is adjusted to pH 3.5 with 0.2N aqueous sodium hydrogen carbonate. The solution is then concentrated to 2 ml under reduced pressure, and purified twice with 30 ml of Diaion HP 10 resin. Fractions containing the desired compound, which are eluted with water, are joined together, and the solvent is distilled off under reduced pressure to obtain 52 mg of white powder. Yield: 70.5%.

IR $\nu_{max}^{cm^{-1}}$ (KBr): 1800, 1790(sh), 1620, (1540 - 1590)

NMR($D_2O$)δ: 6.23(1H, d, J = 5.4Hz), 4.92(1H, d, J = 5.4Hz), 3.9 - 4.3(2H, m), 3.47(3H, s), 2.3 - 2.5 (1H, m), 1.71(1H, ddd, J = 13.8, 4.2, 3.9Hz)

Example 45

Preparation of sodium (±)-cis-7β-phenylacetamido-4α-methoxy-1-azabicyclo[4,2,0]oct-2-en-8-oxo-2-carboxylate:

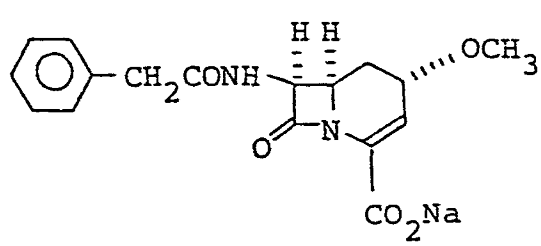

At first, 420 mg of (±)-cis-7β-amino-4α-methoxy-1-azabicyclo[4,2,0]oct-2-en-8-oxo-2-carboxylic acid is dissolved in 80 ml of an aqueous 50% tetrahydrofuran, and the solution is adjusted to pH 7.5 under ice cooling and

stirring by adding powder of sodium hydrogen carbonate thereto. Then, a solution of 2.3 ml of phenylacetyl chloride in 7 ml of tetrahydrofuran is added dropwise thereto at the same temperature, while keeping the pH of the reaction solution at 7.3 - 7.5 with a saturated aqueous sodium hydrogen carbonate solution. The mixture is stirred at the same temperature for one hour, and the precipitate is removed by filtration. The filtrate is concentrated under reduced pressure to distill off tetrahydrofuran. The residue is washed twice with ethyl acetate, and the aqueous layer is adjusted to pH 1.5 with 1N hydrochloric acid and extracted three times with ethyl acetate. After the solvent is distilled off, the residue is dissolved in methanol, and the solution is adjusted to pH 7.8 with a saturated aqueous sodium hydrogen carbonate solution. Then, methanol is distilled off under reduced pressure.

The resulting aqueous solution is passed through a column with 200 ml of Diaion HP 10 resin, and the column is washed with 500 ml of water. Fractions containing the desired compound, which are eluted with water-methanol (10 : 1 V/V), are joined together, and the solvent is distilled off under reduced pressure. The residue is vacuum dried in the presence of phosphorus pentoxide to obtain 480 mg of the desired compound as white powder. Yield: 72.1%.

Physical properties of the compound are given below:

IR $\nu_{max}^{cm^{-1}}$ (KBr): 1770(sh), 1760, 1660, 1600

NMR($CD_3OD$)δ: 7.30(5H, brs), 6.23(1H, d, J = 5.5Hz), 5.35(1H, d, J = 5.4Hz), 3.7 - 4.0(2H, m), 3.60(2H, s), 3.37(3H, s), 1.80 - 2.17(1H, m), 1.27 - 1.67 (1H, m)

Example 46

Preparation of [4S,6R,7S]-7-amino-4-methoxy-1-azabicyclo[4,2,0]oct-2-en-8-oxo-2-carboxylic acid:

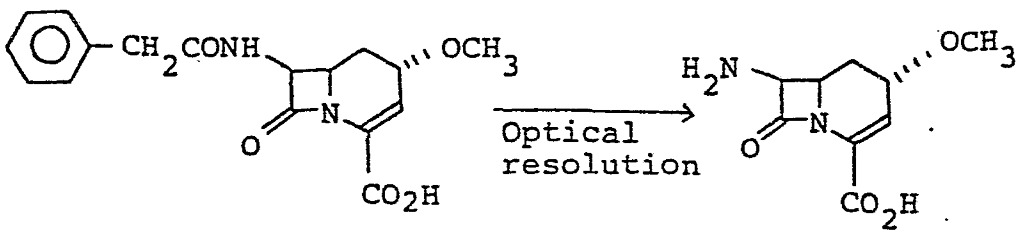


46-1) Preparation of cell suspension

46-2) Preparation of substrate solution

46-3) Enzyme reaction

The same procedures as in Reference Example 41-1), 41-2) and 41-3) are carried out except that 480 mg of sodium (±)-cis-7β-phenylacetamido-4α-methoxy-1-azabicyclo [4,2,0]oct-2-en-8-oxo-2-carboxylate is used.

46-4) Isolation and purification

After completion of the reaction, cells are removed from the reaction mixture by centrifugation, and the supernatant is adjusted to pH 3.0 with 2N hydrochloric acid. Then, the reaction mixture is charged onto a column filled with 200 ml of Diaion HP 10. Fractions containing the desired compound, which are eluted with water, are joined together, concentrated under reduced pressure, and freeze-dried to obtain 107.7 mg of white powder. Yield: 71.1%.

Physical properties of the compound are given below:

$$[\alpha]_D^{20°} = -86.0°$$ (c=0.25, 1 M phosphate buffer, pH 7)

IR and NMR spectra thereof are the same as those of the corresponding dl isomer.

Reference Example 1

Preparation of diphenylmethyl ester of [4S,6R,7S]-7-[2-(2-tritylamino-4-thiazolyl)-2-syn-methoxyiminoacetamido]-4-hydroxy-1-azabicyclo[4,2,0]oct-2-en-8-oxo-2-carboxylic acid:

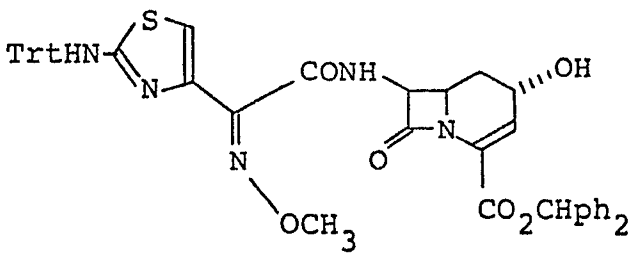

At first, 1.93 g (2.98 m moles) of diphenylmethyl ester of [4S,6R,7S]-7-[2-(2-tritylamino-4-thiazolyl)-2-syn-methoxyiminoacetamido]-4-hydroxy-1-azabicyclo[4,2,0]oct-2-en-8-oxo-2-carboxylic acid is dissolved in 150 ml of water, and 100 ml of ethyl acetate is added thereto. Then, 2N hydrochloric acid is added thereto to adjust the aqueous layer to pH 2. After separation of the ethyl acetate layer, the aqueous layer is extracted four times with 50 ml of ethyl acetate. The organic layers are joined together, washed with a saturated aqueous sodium chloride solution and dried over sodium sulfate. The layer is concentrated under reduced pressure to about 70 ml, and 30 ml of chloroform is added thereto. Diphenyldiazomethane is added thereto until it is not discolored. After stirring for 7.5 hours, deposited crystals are separated by filtration, and washed with ether, whereby 880 mg of yellow powder is obtained. The filtrate is concentrated under reduced pressure, and purified by silica gel column chromatography. The column is developed with n-hexane-ethyl acetate (1 : 1), and fractions containing the desired compound are joined together, and concentrated under reduced pressure to obtain 1.12 g of yellow powder. By joining it with 880 mg of the former powder, 2.00 g (2.53 m moles) of the desired compound is obtained. Yield: 85.0%.

NMR($CDCl_3$)$\delta$: 7.5 - 7.0(27H, m), 6.95(1H, s), 6.56(1H, s), 6.50(1H, d, J = 5.4Hz), 5.43(1H, dd, J = 5Hz, 5Hz), 4.35(1H, m), 4.10(1H, m), 3.96(3H, s), 1.7 - 1.2 (2H, m)

IR $\nu_{max}^{cm^{-1}}$ ($CHCl_3$): 1795, 1782, 1735, 1685

Reference Example 2

Antibacterial effects of the present compounds by dilution method on Mueller-Hinton agar are shown in the following table (MIC µg/ml).

| Micro-organism * | Compound of Exs. 5 and 17 | Compound of Ex. 6 | Compound of Ex. 13 | Compound of Ex.15 | Compound of Ex.23 | Compound of Ex.26 |
|---|---|---|---|---|---|---|
| 1 | 1.56 | 3.13 | 3.13 | 6.25 | 3.13 | 6.25 |
| 2 | 0.1 | 0.05 | 0.78 | 0.1 | 0.39 | 0.05 |
| 3 | 0.02 | 0.02 | 0.39 | 0.02 | 0.2 | 0.02 |
| 4 | 6.25 | 0.05 | 6.25 | 6.25 | 25 | 6.25 |
| 5 | | ≦0.01 | 0.02 | ≦0.01 | ≦0.01 | ≦0.01 |
| 6 | ≦0.01 | 0.05 | 0.78 | ≦0.01 | 0.2 | 0.02 |
| 7 | | ≦0.01 | 0.2 | ≦0.01 | 0.02 | ≦0.01 |
| 8 | 25 | 3.13 | 50 | 50 | 50 | 25 |
| 9 | 6.25 | 0.1 | 12.5 | 12.5 | 12.5 | 12.5 |
| 10 | 12.5 | 0.02 | 25 | 25 | 12.5 | 25 |
| 11 | >100 | 25 | >100 | >100 | >100 | >100 |

* 1 Staphilococcus aureus 209-P
2 Escherichia coli NIHJ C-2
3 Krebsiella pneumoniae 8045
4 Serratia marcescens T-26
5 Proteus vulgaris 6897
6 Proteus morganii 4298
7 Proteus rettgeri 4289
8 Enterobacter aerogenes F-1948
9 Pseudomonas aerginosa 145
10 Pseudomonas sepatia F-2251
11 Pseudomonas maltphilia F-3438

Reference Example 3

Preparation of tertiary butyl ester of (±)-cis-7β-phthalimido-1-azabicyclo[4,2,0]oct-2-en-8-oxo-2-carboxylic acid:

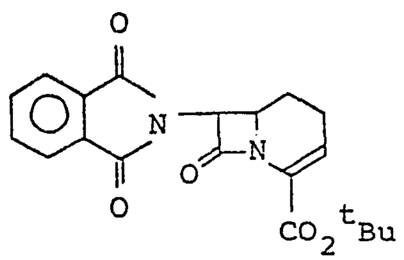

3-1) Preparation of tertiary butyl ester of cis-4-(3,3-dimethoxy-1-propen-1-yl)-3-phthalimido-2-oxoazetidin-1-yl-α-diethylphosphonoacetic acid:

At first, 20 g (74.8 m moles) of tertiary butyl ester of α-amino-α-diethylphosphonoacetic acid is dissolved in 160 ml of ethyl acetate, and 11.43 g (82.3 m moles) of 4,4-dimethyl-trans-2-butenal is added to the solution. The mixture is heated at 50°C for 10 minutes. The solvent is distilled off at a bath temperature of 50°C under reduced pressure, whereby a Schiff base is obtained as oily matters. Then, 250 ml of anhydrous methylene chloride is added to the oily matters, and 12.7 ml (89.8 m moles) of triethylamine is added to the resulting solution under ice cooling and stirring. A solution of 18.4 g (82.3 m moles) of phthalylglycinyl chloride in 60 ml of methylene chloride is added dropwise to the reaction mixture over one hour. After the dropwise addition, the ice-water bath is removed, and the mixture is stirred as such to room temperature for 2 hours. Then, the insoluble matters are removed from the reaction mixture by filtration, and the filtrate is washed with 100 ml of a saturated aqueous sodium chloride solution, twice with a solution consisting of 25 ml of a saturated aqueous sodium hydrogen sulfite solution and 25 ml of water, and then twice with a saturated aqueous sodium chloride solution. The organic layer is dried over anhydrous sodium sulfate, and the solvent is distilled off to obtain 46 g of oily matters (hereinafter referred to as

"crude acetal compound"). The crude acetal compound is purified by silica gel chromatography [Wako gel C-200 : 2 ℓ, elution with n-hexane-ethyl acetate (1 : 2 V/V)], whereby 31.8 g of the desired compound (as a mixture of about 1 : 1 diasteromers) is obtained as crystalline oily matters. Yield: 75%.

NMR($CDCl_3$)δ: 1.30 - 1.53(15H, m), 2.98(3/2H, s), 3.01(3/2H, s), 3.08(3/2H, s), 3.10(3/2H, s), 4.06 - 4.41(4H, m), 4.61(1/2H, d, J = 5Hz), 4.62 (1/2H, d, J = 5Hz), 4.81 - 5.04(1H, m), 4.99(1/2H, d, J = 24Hz), 5.02(1/2H, d, J = 24Hz), 5.51 - 5.74 (2H, m), 5.9 - 6.2(1H, m), 7.68 - 7.92(4H, m)

IR $\nu_{max}^{cm^{-1}}$ ($CHCl_3$): 1790(sh), 1780, 1775, 1730

3 -2) Preparation of tertiary butyl ester of cis-4-(3-oxo-1-propen-1-yl)-3-phthalimido-2-oxoazetidin-1-yl-α-diethylphosphonoacetic acid:

At first, 7.1 g of p-toluenesulfonic acid monohydrate is added to a solution of 46 g of the crude acetal compound obtained in 3-1) in a mixed solvent of 400 ml of methylene chloride and 20 ml of acetone, under ice cooling and stirring, and the mixture is stirred as such for one hour and a half. Then, water is added to the reaction mixture, and the organic layer is washed with water, twice with a saturated aqueous sodium chloride solution, once with a saturated aqueous sodium hydrogen carbonate solution and three times with a saturated aqueous sodium chloride solution. The organic layer is dried over anhydrous sodium sulfate, and the solvent is distilled off to obtain 35 g of oil matters (hereinafter referred to as "crude aldehyde compound). The crude aldehyde compound is purified by silica gel chromatography [Wako gel C-200; 600 ml, elution with n-hexane-ethyl acetate (1 : 1 V/V)], whereby 28.3 g of the desired compound (as a mixture of diasteromers) is obtained as crystalline oil matters. Yield: 72.7%.

Physical properties of the compound are given below:

IR $\nu_{max}^{cm^{-1}}$ $(CHCl_3)$: 1790(sh), 1780, 1730, 1695

NMR$(CDCl_3)\delta$: 9.5(1H, d, J = 8.0Hz), 7.8(4H, br), 6.9 - 7.4(1H, m), 5.9 - 6.3(1H, m), 5.7(1H, m), 5.1(1H, d, J = 23Hz), 4.0 - 4.5(5H, m), 1.5(9H, s), 1.4(6H, t, J = 7Hz)

3 -3) Preparation of tertiary butyl ester of (±)-cis-7β-phthalimido-1-azabicyclo[4,2,0]oct-2-en-8-oxo-2-carboxylic acid:

At first, 60 g of tertiary butyl ester of cis-4-(3-oxo-1-propen-1-yl)-3-phthalimido-2-oxoazetidin-1-yl-α-diethylphosphonoacetic acid obtained in 3 -2) is dissolved in 600 ml of dimethoxyethane, and 30 g of 5% palladium carbon is added thereto. Then, a hydrogen gas is introduced in the mixture at 40°C with stirring for 3 hours. After completion of the reaction, the catalyst is removed by filtration, and 20 g of diazabicyclooctane is added to the reaction mixture. The mixture is allowed to stand at room temperature overnight. Then, 1 ℓ of chloroform is added to the reaction mixture, and the mixture is washed with dilute hydrochloric acid and water, and concentrated under reduced pressure. The residue is treated with ethyl acetate, whereby colorless needle-like crystals are obtained. By filtration, 29.6 g of the desired compound is obtained. Yield: 70%.

Physical properties of the compound are given below:

IR $\nu_{max}^{cm^{-1}}$ $(CHCl_3)$: 1800, 1780, 1735, 1635

NMR$(CDCl_3)\delta$: 7.82(4H, m), 6.35(1H, m), 5.64(1H, d, J = 5.0Hz), 3.88(1H, m), 1.67 - 2.58(4H, m), 1.57(9H, s)

Reference Example 4

Preparation of [4S,6R,7S]-7-[2-(2-aminothiazol-4-yl)-2-syn-methoxyiminoacetamido]-4-acetoxy-1-azabicyclo[4,2,0]oct-2-en-8-oxo-2-carboxylic acid:

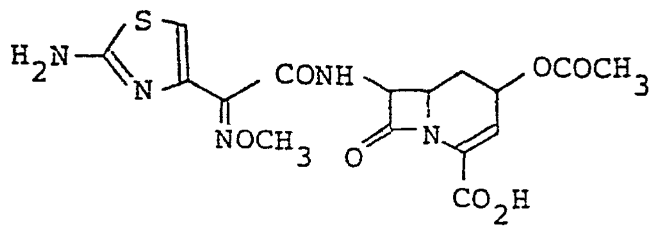

At first, 20 g (31 m moles) of sodium [4S,6R,7S]-7-[2-(2-tritylamino-4-thiazolyl)-2-syn-methoxyiminoacetamido]-4-hydroxy-1-azabicyclo[4,2,0]oct-2-en-8-oxo-2-carboxylate prepared in the process of Example 13 of Japanese Published Patent Application No. 118084/81 is suspended in 600 ml of pyridine, and 29.3 ml (310 m moles) of acetic anhydride is added thereto. The mixture is stirred at room temperature for 3.5 hours. Then, 10 ml (106 m moles) of acetic anhydride is further added thereto, and the mixture is further stirred at room temperature for 8 hours. The reaction mixture is concentrated under reduced pressure, and subjected twice to azeotropic distillation with toluene. Then, 600 ml of ether is added thereto, and the deposited precipitate is recovered by filtration, washed with ether and dried to obtain 21.2 g of yellow powder. The yellow powder is suspended in 500 ml of aqueous 50% acetic acid, and the suspension is stirred at 50°C for 1.5 hours. Then, the reaction mixture is concentrated under reduced pressure, and subjected twice to azeotropic distillation with water. The residue is dissolved in dimethylsulfoxide, and purified with 650 ml of Diaion HP 10 resin. After washing with 1700 ml of water, 1350 ml of water-methanol (8 : 1), 1200 ml of water-methanol (5 : 1) and 1200 ml of water-methanol (3 : 1), fractions eluted with water-methanol (2 : 1) are joined together, and concentrated under reduced pressure. The residue is dissolved in 150 ml of methanol, and 600 ml of ether is added to the solution. The deposited precipitate is separated by filtration, washed with ether and vacuum-dried to obtain 8.34 g (19.7 m moles) of the desired compound as yellow powder. Yield: 63.6%.

NMR($CD_3OD$)δ: 6.84(1H, s), 6.37(1H, d, J = 5.1Hz), 5.61(1H, d, J = 5.4Hz), 5.6 - 5.2(1H, m), 4.1 - 3.9 (1H, m), 3.96(3H, s), 2.07(3H, s), 2.1 - 1.8(2H, m)

IR $\nu_{max}^{cm^{-1}}$ (KBr): 3500, 1780, 1675, 1650

Reference Example 5

Preparation of 2-(2-tritylamino-4-thiazolyl)-2-syn-(3,4-diacetoxybenzoyloxyimino) acetic acid:

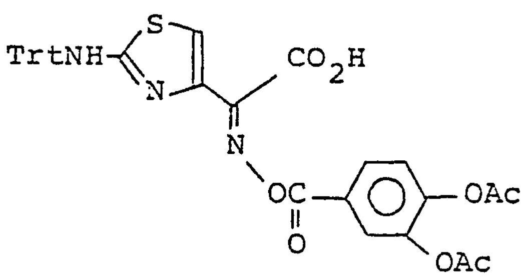

The compound is prepared according to the following steps 1) - 3).

1) Preparation of diphenylmethyl 2-(2-tritylamino-4-thiazolyl)-2-syn-hydroxyiminoacetate:

At first, 8.58 g of 2-(2-tritylamino-4-thiazolyl)-2-syn-hydroxyiminoacetic acid is suspended in 100 ml of tetrahydrofuran, and diphenyldiazomethane is added thereto at room temperature with stirring until the red color of the reaction mixture does not disappear. Then, 1 ml of acetic acid is added thereto, and the mixture is diluted with ethyl acetate. The organic layer is washed with a saturated aqueous sodium chloride solution, a saturated aqueous sodium hydrogen carbonate solution and a saturated aqueous sodium chloride solution in this order, dried over sodium sulfate, and concentrated under reduced pressure to obtain a crude ester. The ester is purified by silica gel chromatography [Wako gel C-100 (made by Wako Junyaku Co., Ltd., Japan), elution with n-hexane-ethyl acetate (3 : 1 V/V)], whereby 6.07 g of the ester is obtained. Yield: 51%.

0082501

NMR($CDCl_3$)δ: 7.13(25H, m), 7.07(1H, s), 6.10(1H, s)

2) Preparation of diphenylmethyl 2-(2-tritylamino-4-thiazolyl)-2-syn-(3,4-diacetoxybenzoyloxyimino) acetate:

At first, 910 mg of diphenylmethyl 2-(2-tritylamino-4-thiazolyl)-2-syn-hydroxyiminoacetate is dissolved in 10 ml of anhydrous methylene chloride, and 0.230 ml of triethylamine is added thereto under ice cooling and stirring.

Separately, 385.6 mg of 3,4-diacetoxybenzoic acid is suspended in 3 ml of anhydrous methylene chloride, 337 mg of phosphorus pentachloride is added to the suspension with stirring to undergo reaction for 30 minutes, and the mixture is concentrated under reduced pressure to obtain an acid chloride.

The acid chloride is added to the first mixture, and the mixture is subjected to reaction for one hour, and then diluted with 20 ml of chloroform, washed with a saturated aqueous sodium hydrogen carbonate solution, water and a saturated aqueous sodium chloride solution in this order, dried over sodium sulfate and concentrated under reduced pressure to obtain 1.13 g of desired 3,4-diacetoxybenzoyl compound.

NMR($CDCl_3$)δ: 7.5 - 7.1(27H, m), 7.0(1H, d, J = 9Hz), 6.80(1H, s), 2.30(3H, s), 2.27(3H, s)

3) Preparation of 2-(2-tritylamino-4-thiazolyl)-2-syn-(3,4-diacetoxybenzoyloxyimino) acetic acid:

At first, 1.13 g of diphenylmethyl 2-(2-tritylamino-4-thiazolyl)-2-syn-(3,4-diacetoxybenzoyloxyimino) acetate is dissolved in 5 ml of anhydrous methylene chloride and 5 ml of anhydrous nitromethane, and a solution of 880 mg of aluminum chloride in 4 ml of anhydrous nitromethane is added thereto dropwise under ice cooling and stirring. The reaction mixture is diluted with 50 ml of ethyl acetate 20 minutes after the dropwise addition, washed once with 1N hydrochloric acid and twice with a saturated aqueous sodium chloride solution, dried over sodium sulfate and

0082501

concentrated under reduced pressure. Then, 20 ml of toluene is added to the residue, and subjected to disintegration. The toluene layer is removed by decantation, whereby 649 mg of the carboxylic acid is obtained. Then, an equivalent amount of n-hexane is added to the toluene layer, and the deposited precipitate is recovered, whereby further 100 mg of the carboxylic acid is obtained.

NMR($DMSO-d_6$)δ: 7.9 - 7.8(2H, m), 7.6 - 7.1(17H, m), 2.27(6H, s)

IR $\nu_{max}^{cm^{-1}}$ (KBr): 3435, 1790, 1775(sh), 1750(sh), 1600

Reference Example 6

Preparation of 2-(2-tritylamino-4-thiazolyl)-2-syn-(3,4-diacetoxybenzoyloxyimino) acetic acid (another process):

At first, 4.29 g of 2-(2-tritylamino-4-thiazolyl)-2-syn-hydroxyiminoacetic acid is suspended in 50 ml of anhydrous methylene chloride, and 2.77 ml of triethylamine is added thereto under ice cooling and stirring to dissolve the acid. A solution in 10 ml of anhydrous methylene chloride of the acid chloride prepared from 2.34 g of 3,4-diacetoxybenzoic acid in the same manner as in Ref. Ex. 5-2) is added dropwise to the solution. The reaction mixture is washed, one hour after the dropwise addition, once with 1N hydrochloric acid, and twice with a saturated aqueous sodium chloride solution, dried over sodium sulfate, and concentrated under reduced pressure to obtain crude carboxylic acid. The residue is disintegrated in ether, stirred in ether overnight, and recovered by filtration, whereby 5.31 g of the desired carboxylic acid is obtained.

IR and NMR of the compound are identical with those obtained in Reference Example 5-3).

Reference Example 7

Preparation of 2-(2-formylamino-4-thiazolyl)-2-syn-(3,4-diacetoxybenzoyloxyimino) acetic acid:

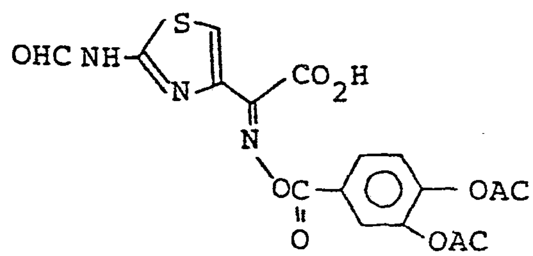

At first, 215 mg of 2-(2-formylamino-4-thiazolyl)-2-syn-hydroxyiminoacetic acid and 306 µl (2.2 m moles) of triethylamine are dissolved in 4 ml of dichloromethane, and a solution of 256 mg (1 m mole) of 3,4-diacetoxybenzoyl chloride in 3 ml of dichloromethane is added thereto under ice cooling and stirring. The mixture is stirred under ice cooling for 30 minutes and at room temperature for 30 minutes, and then the reaction mixture is poured into water. The mixture is adjusted to pH 1 with 10% hydrochloric acid and extracted twice with ethyl acetate. The extract is washed with an aqueous sodium chloride solution, dried over sodium sulfate, and concentrated under reduced pressure. The residue is disintegrated to powder in ethyl acetate-hexane (1 : 1) and recrystallized from chloroform-methanol, whereby 150 mg of the desired compound is obtained.

NMR($DC_3OD$)δ: 8.57(1H, s), 8.2 - 7.7(2H, m), 7.5 - 7.3 (2H, m), 3.3(6H, s)

IR $\nu_{max}^{cm^{-1}}$ (KBr): 3150(br), 1795, 1782, 1775, 1750, 1559

Reference Example 8

Preparation of (S)-2-(3,4-diacetoxybenzamido)-2-(2-chloroacetamido-4-thiazolyl) acetic acid:

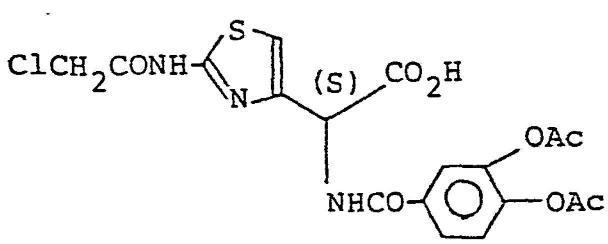

At first, 1 ml of an aqueous 0.2N sodium hydrogen carbonate solution is added to 50 mg of (S)-2-amino-2-(2-chloroacetamido-4-thiazolyl) acetic acid, and the mixture is heated to dissolve the acid and then ice-cooled. A solution of 77 mg of 3,4-diacetoxybenzoyl chloride in 0.5 ml of acetone is added thereto, and the reaction mixture is adjusted to pH 7.0 - 7.2 with an aqueous 0.4N sodium hydrogen carbonate solution.

The mixture is stirred under ice cooling for 30 minutes and at room temperature for 30 minutes, adjusted to about pH 1 with 1N hydrochloric acid, and extracted three times with ethyl acetate. The organic layers are joined together, washed three times with a saturated aqueous sodium chloride solution, and dried over sodium sulfate. After sodium sulfate is removed by filtration, the filtrate is concentrated under reduced pressure to obtain oily matters. Then, 2 ml of chloroform is added to the oily matters, and the deposited crystals are separated by filtration and dried to obtain 55 mg of the desired compound. Yield: 58.5%.

NMR($CDCl_3$-$CD_3OD$)δ: 7.80 - 7.20(3H, m), 7.13(1H, s), 5.83(1H, s), 4.23(2H, s), 2.30(6H, s)

Reference Example 9

Synthesis of [4S,6R,7S]-7-[2-(2-aminothiazol-4-yl)-2-amino] acetamido-4-acetoxy-1-azabicyclo[4,2,0]oct-2-en-8-oxo-2-carboxylic acid hydrochloride:

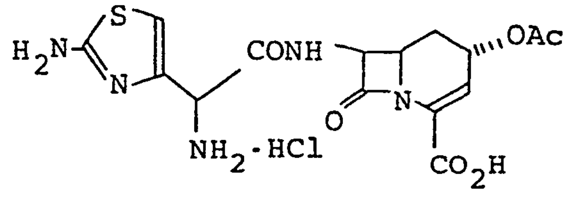

At first, 500 mg (1.18 m moles) of [4S,6R,7S]-7-[2-(2-aminothiazol-4-yl)-2-syn-methoxyimino] acetamido-1-azabicyclo[4,2,0]oct-2-en-8-oxo-2-carboxylic acid is dissolved in 5 ml of water adjusted to pH 0.8 with 2N hydrochloric acid, and 400 mg of zinc powder is added thereto. The mixture is stirred at room temperature for 2 hours while keeping the mixture at pH 1 with 2N hydrochloric acid. After removal of the zinc powder by filtration, the filtrate is subjected to column chromatography with 60 ml of Diaion HP-10. After washing with 130 ml of water, the fractions eluted with water : methanol (8 : 1 - 6 : 1) and colored with ninhydrin are collected and concentrated under reduced pressure to obtain 298 mg (0.64 m moles) of the desired compound as yellow solid matters.

NMR($D_2O$) δ ppm: 6.90(1H, s), 6.05(1/2H, d, J = 5.5Hz), 6.02(1/2H, d, J = 5.5Hz), 5.6 - 5.2(2H, m), 5.15 (1H, bs), 4.2 - 3.8(1H, m), 2.07(3H, s), 2.2 - 1.3 (2H, m)

IR (KBr) $\nu_{max}^{cm^{-1}}$ : 3350, 1780(s), 1770, 1760, 1600, 1520

Reference Example 10

Synthesis of [4S,6R,7S]-7-[2-(2-aminothiazol-4-yl)-2-(3,4-diacetoxybenzamido)] acetamido-4-acetoxy-1-azabicyclo[4,2,0]oct-2-en-8-oxo-2-carboxylic acid:

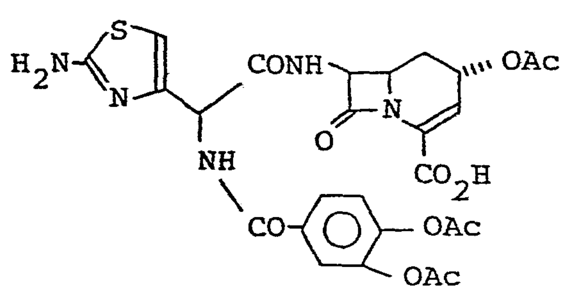

At first, 274 mg (0.585 m moles) of [4S,6R,7S]-7-[2-(2-aminothiazol-4-yl)-2-amino] acetamido-4-acetoxy-1-azabicyclo[4,2,0]oct-2-en-8-oxo-2-carboxylic acid hydrochloride is dissolved in 2 ml of water and 4 ml of tetrahydrofuran, and 50 μl of triethylamine is added thereto at room temperature with stirring to make pH 7.0. Then, a solution of 150 mg of 3,4-diacetoxybenzoyl chloride in 2 ml of tetrahydrofuran is added thereto, while keeping the

reaction mixture at pH 6 - 7 with triethylamine. After stirring at room temperature for 40 minutes, 40 mg of 3,4-diacetoxybenzoyl chloride is added thereto in the same manner as above.

After distilling off tetrahydrofuran by concentration under reduced pressure, the residues are adjusted to pH 2 with 2N hydrochloric acid and the mixture is stirred under ice cooling for 15 minutes. The deposited yellowish white precipitates are separated by filtration, washed with water, and dried to obtain 283 mg (0.471 m mole) of the desired compound as yellowish white powder.

NMR($CD_3OD$) $\delta$ ppm: 7.95 - 7.70(2H, m), 7.30(1H, d, J = 8Hz), 6.60(1H, s), 6.33(1/2H, d, J = 5.5Hz), 6.30(1/2H, d, J = 5.5Hz), 5.62(1/2H, s), 5.58 (1/2H, s), 5.6 - 5.3(2H, m), 2.28(6H, s), 2.02(3H, s)

IR (KBr) $\nu_{max}^{cm^{-1}}$: 3300, 1785(s), 1775, 1640, 1525, 1495

What is Claimed is:

1. A β-lactam compound represented by the general formula:

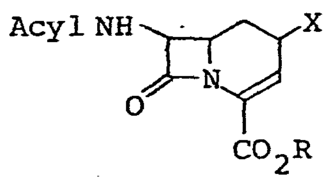

{wherein Acyl represents:

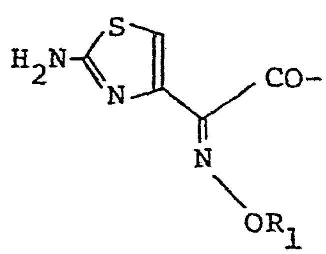

[where $R_1$ represents a hydrogen atom, a lower alkyl group having 1 to 6 carbon atoms, a substituted lower alkyl group having 1 to 6 carbon atoms (where the substituent is a cyano group, a halogen atom, a carboxyl group, a lower alkoxycarbonyl group having 2 to 6 carbon atoms, or a carbamoyl group), or a group represented by:

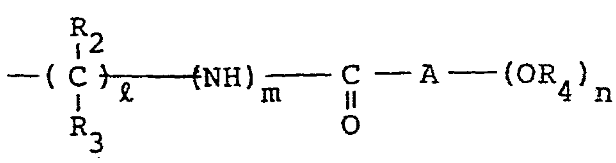

(where $R_2$ and $R_3$ are same or different, and represent hydrogen atoms, lower alkyl groups having 1 to 5 carbon atoms, or a 3 to 6-membered cycloalkylidene group with the carbon atoms bonded thereto; A represents a phenyl or naphtyl group respectively substituted by $(OR_4)_n$ and further

optionally substituted by other group(s); $R_4$ represents a hydrogen atom, or a lower alkanoyl group having 1 to 5 carbon atoms; ℓ and m represent 0 or 1; n represents an integer of 1 to 4)], or

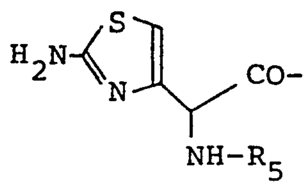

[where $R_5$ represents a hydrogen atom, a lower alkanoyl group having 1 to 5 carbon atoms, a carbamoyl group, a lower alkyl group having 1 to 5 carbon atoms, a carbamoyl group substituted by a lower alkanoyl group having 1 to 5 carbon atoms, or a group represented by:

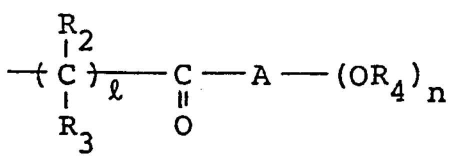

(where $R_2$, $R_3$, A, $R_4$, ℓ and n have the same meanings as defined above)]; X represents a methoxy group, an azide group, an amino group, $NHCOR_6$ (where $R_6$ represents a lower alkyl group having 1 to 5 carbon atoms, an amino group, or a lower alkylamino group having 1 to 5 carbon atoms, $S-R_7$ (where $R_7$ represents a substituted or unsubstituted 5 or 6-membered monocyclic or bicyclic heterocyclic group having 1 to 5 nitorgen, oxygen or sulfur atoms), a pyridinium group, or a substituted pyridinium group; and R represents a hydrogen atom, an alkali metal, an alkaline earth metal, an organic ammonium group or an ester residue readily hydrolyzable in vivo. In spite of the above definition, when X is a methoxy group, $R_1$ is a hydrogen atom, a lower alkyl group having 1 to 6 carbon atoms, or a substituted lower alkyl group having 1 to 6 carbon atoms (where the substituent is a cyano group, a halogen atom, or a carbamoyl group) } and a pharmacologically acceptable acid or base addition salt thereof.

2. A compound according to claim 1 wherein Acyl is

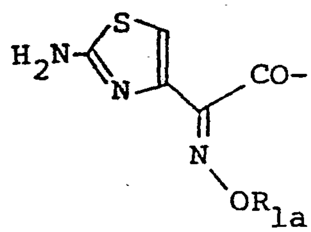

[where $R_{1a}$ represents a lower alkyl group having 1 to 6 carbon atoms, a lower alkyl group having 1 to 6 carbon atoms substituted by a carboxyl group, or

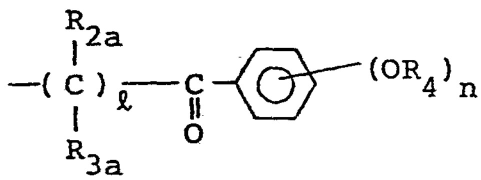

(wherein $R_{2a}$ and $R_{2b}$ are same or different, and represent hydrogen atoms or lower alkyl groups having 1 to 5 carbon atoms, and $R_4$, ℓ and n have the same meanings as defined above)], or

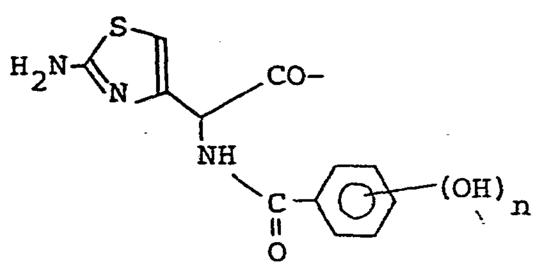

(where n has the same meaning as defined above); and X is an azido group, an amino group, $-NHCOR_6$ (where $R_6$ has the same meaning as defined above), $-S-R_{7a}$ [where $R_{7a}$ represents a 5 or 6-membered monocyclic or bicyclic heterocyclic group having 1 to 4 nitrogen, or 1 to 3 nitrogen and one sulfur atoms optionally substituted by a lower alkyl group having 1 to 5 carbon atoms, an amino group, a phenyl group, $-(CH_2)_qCO_2H$ (where q has the same meaning as defined above) or a phenyl group], or a pyridinium group.

3. A compound according to claim 2 wherein said heterocyclic group in respect of $R_{7a}$ is selected from a pyrrolyl group, an imidazolyl group, a pyrazinyl group, a thiazolyl group, an isothiazolyl group, a thiadiazolyl group, a triazolyl group, a tetrazolyl group, a pyridinyl group, a pyrimidinyl group, a triazinyl group and a benzothiazolyl group.

4. A compound according to claim 2 wherein R is a hydrogen atom, an alkali metal or an alkaline earth metal.

5. A β-lactam compound represented by the general formula:

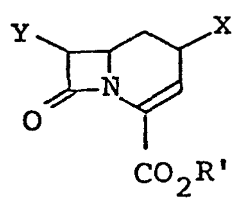

(wherein Y represents an amino group, a phthalimido group or a phenylacetamido group; X has the same meaning as defined above; and R' has the same meaning as R defined above or further represents a carboxyl-protecting group) and an acid addition salt thereof in case of Y being an amino group.

6. A compound according to claim 5 wherein said carboxyl-protecting group is selected from a t-butyl, benzyl, p-nitrobenzyl, benzhydryl, trityl and trialkylsilyl group.

7. An antimicrobial pharmaceutical composition which comprises an effective antimicrobial amount of a compound according to any of claims 1 to 4 and at least one member of the group consisting of pharmaceutically acceptable diluents, carriers and adjuvants.

0082501

European Patent Office

# EUROPEAN SEARCH REPORT

Application number

EP 82 11 1736

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | GB-A-2 017 102 (KYOWA) *Claims* | 1-7 | C 07 D 471/04<br>A 61 K 31/435//<br>C 07 F 9/65<br>C 07 D 277/42<br>(C 07 D 471/04<br>C 07 D 221/00<br>C 07 D 205/00 ) |
| | --- | | |
| D,X | EP-A-0 025 602 (KYOWA) *Claims* | 1-7 | |
| | --- | | |
| D,X | EP-A-0 027 882 (KYOWA) *Claims* | 5,6 | |
| | --- | | |
| Y | CHEMICAL ABSTRACTS, vol. 96, no. 19, 10th May 1982, page 729, no. 162430z, Columbus Ohio (USA); & JP - A - 81 152 479 (KYOWA HAKKO KOGYO CO., LTD.) (26-11-1981) *Abstract* | 1-4,7 | |
| | --- | | TECHNICAL FIELDS SEARCHED (Int. Cl. 3) |
| Y | CHEMICAL ABSTRACTS, vol. 95, no. 21, 23rd November 1981, page 640, no. 187101k, Columbus Ohio (USA); & JP - A - 81 49 381 (KYOWA HAKKO KOGYO CO., LTD.) (02-05-1981) *Abstract* | 1-4,7 | C 07 D 471/00<br>A 61 K 31/00 |
| | ----- | | |

The present search report has been drawn up for all claims

| Place of search | Date of completion of the search | Examiner |
|---|---|---|
| THE HAGUE | 14-03-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82